# EUROPEAN PATENT SPECIFICATION

(11) **EP 2 229 980 B1**
(45) Date of publication and mention of the grant of the patent: **12.08.2015**
(21) Application number: 10250491.7
(22) Date of filing: 16.03.2010
(51) Int. Cl.: A61N 5/06, A61N 5/067

(54) **Treatment of microbiological pathogens in a toe nail with antimicrobial light**
Behandlung mikrobiologischer Krankheitserreger in einem Zehennagel mit antimikrobiellem Licht
Traitement de pathogènes microbiologiques dans un ongle d'orteil au moyen de lumière antimicrobienne

(30) Priority: 16.03.2009 US 160400 P; 23.07.2009 US 228126 P
(43) Date of publication of application: 22.09.2010
(73) Proprietor: Nuvolase, Inc., Chico CA 95973 (US)
(72) Inventor: Harris, David M, Linnwood, WA 98036 (US); Strisower, John, Chico, CA 95973 (US); Campbell, Preston, 1906, Reston, VA 20190 (US)
(74) Representative: Carter, Stephen John

(56) References cited:
- WO-A2-2006/125231
- WO-A2-2008/089344
- US-A1- 2003 004 499
- US-A1- 2006 212 025
- US-A1- 2008 077 199

## Description

### BACKGROUND

Infectious diseases are a worldwide problem causing millions of deaths each year. According to the National Institute of Allergy and Infectious Diseases, National Institutes of Health, 13.5 million people died in 1999 from the leading ten infectious causes of death worldwide. These ten diseases, in order of causes of death worldwide, are acute lower respiratory infections, HIV/AIDS, diarrheal diseases, tuberculosis, malaria, measles, tetanus, pertussis, sexually transmitted diseases (excluding HIV), and meningitis. Although vaccines and treatments exist for some of these diseases and their symptoms, many of the diseases do not have appropriate prevention and/or treatment.

There are a variety of types of infectious disease pathogens, including bacterium, viruses (*e*.*g*. rotavirus, which is the leading single cause of severe diarrhea among infants and young children), fungus (*e*.*g*., Apophysomyces elegans, which is responsible for subcutaneous zygomycosis (Mucormycosis)), and parasites (*e*.*g*., Trypanosoma cruzi parasites). These types of pathogens are responsible for various infectious diseases that inflict humans and other living creatures.

Existing approaches to combating infectious disease pathogens typically involve the use of an antimicrobial substance, such as antibiotics (generally used to treat bacterial infections), antiviral drugs (a class of medication used specifically for treating viral infections), antifungal drugs, and antiparasitics. However, in some cases, combating pathogens using antimicrobial substances can be less than fully effective.

The effectiveness of a treatment with an antimicrobial substance can be reduced when the target pathogen(s) develop drug resistance. For example, some bacterial pathogens have developed antibiotic resistance. Antibiotic-resistant enterococci have bedeviled infectious disease treating professionals for decades. Clinical infections caused by enterococcus include urinary tract infections, bacteremia, bacterial endocarditis, diverticulitis, and meningitis. Of all aerobic gram-negative bacilli, E. coli and Klebsiella species most frequently cause disease in humans, and are highly resistant to antibiotic treatment. Bacteremia, hospital-acquired pneumonia, postoperative meningitis, and other nosocomial infections produce life-threatening diseases and have an urgent need for new effective treatment. P. aeruginosa is an invasive, gram-negative bacterial pathogen that causes a wide range of severe infections. Life-threatening infection may occur in patients who become immunocompromised after chemotherapy for cancer or immunosuppressive therapy for organ transplantation. Furthermore, P. aeruginosa causes serious infections of the lower respiratory tract, the urinary tract, and wounds in younger and older hospitalized patients. P. aeruginosa has a greater ability than most gram-positive and many gram-negative pathogens to develop resistance to virtually any antibiotic to which it is exposed, because of multiple resistance mechanisms that can be present within the pathogen concurrently. In some clinical isolates, resistance occurs to all available FDA-approved antibiotics.

Existing antiviral drugs are designed to help with a limited number of viruses, such as Human Immunodeficiency Virus (HIV), herpes viruses (best know for cold sores and genital herpes), hepatitis B and C viruses, and influenza A and B viruses. Although research is underway to extend the range of antiviral drugs to other families of viruses, viruses, like antibiotics, may evolve to resist particular antiviral drugs.

Some existing antifungal drugs often have side-effects, some of which can be life-threatening if the drug is not used properly. Antifungal drugs work by exploiting the differences between the host's cells and the fungal cells to kill the fungus without damaging the host. However, because fungal cells and human cells are similar at the molecular level, it is difficult to find antifungal drugs that do not damage human cells to some extent.

Toenail fungus (onychomycosis) is an example of a fungal disease that offers a challenge for treatment. Infection is often embedded within the nail and is difficult to reach. The disease affects as much as 8% of the general population, and treatment is challenging, complicated by low cure rates and relatively high relapse rates. Without treatment, toenails can become thick, causing pressure, irritation, and pain. This pain can limit physical mobility and activity. Onychomycosis can also impact a patient's quality of life. Many patients report social embarrassment related to the disease. Anxiety, depression, loss of self-esteem and confidence, avoidance of intimacy, and impaired relations are among the negative impacts that have been reported. Current treatments for onychomycosis include oral, topical, and surgical options. All of these, however, require prolonged treatment (at least one year in many cases), and have limited effectiveness.

Accordingly, there is a need for new treatments for combating infectious disease pathogens, particularly when the pathogens are resident within a host, such as a human host.

US-A-2003/0004499 discloses a system and method for the photomodulation of living tissue with application to non-dermatological medical treatments including tumor growth inhibition, cell regeneration, the stimulation of tissue in organs, etc.

WO-A-2006/125231 discloses a photon therapy device providing a controller unit for controlling the emission of at least one photon emitting source, the controller unit including a central processing unit pre-programmed with selectable pre-programmed photonic emission protocols.

US-A-2008/0077199 discloses a method and apparatus for light therapy, which is a method using light for treating diseases and/or maintaining the health of a living organism.

### SUMMARY

The following presents a simplified summary of some embodiments of the present disclosure in order to provide a basic understanding of the invention. This summary is not an extensive overview of the invention. It is not intended to identify key/critical elements of the invention or to delineate the scope of the invention. Its sole purpose is to present some embodiments of the invention in a simplified form as a prelude to the more detailed description that is presented later. The invention is as defined in the appended claims.

In one aspect, a method is disclosed of treating nail disease in a patient. The method includes identifying a nail with a nail disease, aligning a light applicator with a first location on the nail, irradiating the nail and the nail disease with light from the light applicator, and moving the light applicator to other locations on the nail to irradiate the other locations so as to treat the nail disease. The light includes a wavelength corresponding to an absorption spectrum of the nail disease and comprises a duty cycle with a series of alternating light emissions and pauses. The light emissions can include a series of pulses.

Suitable time periods of the emissions and the pauses can be employed in the method. For example, the time periods of the emissions and the pauses can be uniform and/or identical. The time periods of the emissions and the pauses can be approximately one-half second each. The time periods of the emissions can be approximately one-third second each and the time periods of the pauses can be approximately one-sixth second each.

In another aspect, a method is disclosed of treating nail disease in a patient. The method includes identifying a nail with a nail disease, and irradiating the nail and the nail disease with light from the light applicator. The light includes a wavelength corresponding to an absorption spectrum of the nail disease and has a sufficiently short duration and high peak energy such that thermal accumulation is limited within the host tissue to treat the nail disease and limit pain to the patient.

Suitable light parameters can be employed in the method. For example, the light can be pulsed. The duration can be less than a second (*e*.*g*., one-half second, one-third second).

In another aspect, and according to the invention, a system is disclosed for treating nail disease in a patient. The system includes a light applicator operable to irradiate a nail having a nail disease with light that includes a wavelength corresponding to an absorption spectrum of the nail disease and comprising a duty cycle with a series of alternating light emissions and pauses, a light source coupled with the light applicator for supplying the light to the light applicator, and a controller coupled with the light source for controlling the operation of the light source such that the pauses are timed for movement of the light applicator between light emissions. The light emissions can include a series of pulses.

Suitable time periods of the emissions and the pauses can be employed in the system. For example, the time periods of the emissions and the pauses can be uniform and/or identical. The time periods of the emissions and the pauses can be approximately one-half second each. The time periods of the emissions can be approximately one-third second each and the time periods of the pauses can be approximately one-sixth second each.

In another aspect, a system is disclosed for treating nail disease in a patient. The system includes a light applicator operable to irradiate a nail having a nail disease with light that includes a wavelength corresponding to an absorption spectrum of the nail disease and having a sufficiently short duration and high peak energy such that thermal accumulation is limited within the host tissue to treat the nail disease and limit pain to the patient, a light source coupled with the light applicator for supplying the light to the light applicator, and a controller coupled with the light source for controlling the operation of the light source.

Suitable light parameters can be employed in the system. For example, the light can be pulsed. The duration can be less than a second (*e*.*g*., one-half second, one-third second).

In another aspect, a method for determining a treatment for combating a pathogen disposed within a host is provided. The method includes selecting a pathogen, selecting a host tissue, selecting a light that includes a wavelength that corresponds to an absorption spectrum of the pathogen and that can at least partially penetrate the host tissue, selecting treatment parameters for the delivery of the light into the host tissue, and selecting a delivery mechanism for irradiating the host tissue with the light.

A method for determining a treatment for combating a pathogen disposed within a host can involve a number of options. For example, the light can include wavelengths between 410 nm and 10.6 microns, and more specifically between 550 nm and 1800 nm, and even more specifically around 1064 nm. The irradiating can include a pulsed application of laser light. The pulsed application can include 200 mJ pulses. The irradiating can include one or more bursts of pulsed application of laser light. The laser light can be pulsed, for example, at 20 Hz and the bursts can be applied at 1 Hz. The pathogen can reside within various locations, such as within a patient's toe, foot, leg, or arm. The pathogen can reside within or adjacent to a patient's auditory system tissues, sinus cavity tissues, vocal cords, tissues adjacent to respiratory track cavities, tissues adjacent to digestive track cavities, reproductive system tissues, urinary system tissues, urinary track, or bladder. The pathogen can reside within a bone.

In another aspect, a method for treating toenail fungus (*e*.*g*., onychomycosis) is provided. The method includes identifying a toenail with toenail fungus, and irradiating the toenail and the toenail fungus with laser light that includes a wavelength corresponding to an absorption spectrum of the toenail fungus.

Single spots can be irradiated on an infected toenail, with each of the spots being approximately 1 mm or larger in diameter. A 0.22 numerical aperture (NA) fiber tip for the laser can be held approximately 3 mm from the nail surface. The nail surface can be irradiated with the pulse laser light having an energy of 200 mJ or greater per pulse. The pulse laser light can penetrate the target tissue 1 mm or greater. The wavelength of the pulse laser light according to the invention is approximately 1064 nanometers. The pulse duration can be 80 microseconds, the pulse frequency according to the invention is 10 to 30 hertz, and the burst frequency can be approximately 1 Hz.

In another aspect, a method for determining a treatment for combating a blood-borne pathogen is provided. The method includes selecting a blood-borne pathogen, selecting light that includes a wavelength that corresponds to an absorption spectrum of the pathogen and that can at least partially penetrate a patient's blood, selecting treatment parameters for delivery of the light into a patient's blood; and selecting a delivery mechanism for irradiating a patient's blood with the light from within a blood vessel, wherein the irradiating is coordinated with the flow of blood within the blood vessel. The irradiating can be accomplished by varying the intensity of the light in coordination with the flow of blood. The irradiating can be accomplished by a pulsed application of the light in coordination with the flow of blood.

In another aspect, a method for determining a treatment for combating an air-borne pathogen is provided. The method includes selecting an air-borne pathogen, selecting a host air passage, selecting light that includes a wavelength that corresponds to an absorption spectrum of the pathogen, selecting treatment parameters for the delivery of the light from within the host air passage, and selecting a delivery mechanism for irradiating the pathogen with the light from within the host air passage. The host air passage can be a patient's trachea. The selected light can include ultraviolet light and host tissue can be substantially shielded from irradiation.

In another aspect, a system for internally applying an antimicrobial-light treatment is provided. The system includes a light applicator integrated with an endoscope, a light source coupled with the light applicator for supplying antimicrobial light to the light applicator, and a controller coupled with the light source for controlling the operation of the light source. The light source can generate a light with a wavelength that corresponds to an absorption spectrum of a pathogen and that can at least partially penetrate a tissue of the patient.

In another aspect, a system for externally applying penetrating-antimicrobial light to a patient's tissue is provided. The system includes a light applicator that includes an opening in which the patient's tissue can be placed, a light source coupled with the light applicator for supplying the penetrating-antimicrobial light to the light applicator, and a controller coupled with the light source for controlling the operation of the light source. The light applicator directs penetrating-antimicrobial light towards the patient's tissue from multiple directions. The light applicator can include a plurality of light emitters. The system can be configured to vary the emission of light between the emitters. The system can be configured to vary at least one direction along which the penetrating-antimicrobial light is directed. The system can be configured to vary a focal point for the penetrating-antimicrobial light.

In another aspect, a system for combating an airborne pathogen leaving and/or prior to entering the respiratory system of a patient is provided. The system includes an interface component configured for coupling with a patient's respiratory system, a light applicator coupled with the interface component, a light source coupled with the light applicator for supplying antimicrobial light to the light applicator, and a controller coupled with the light source for controlling the operation of the light source. The light applicator is configured to apply antimicrobial light to airborne pathogens transferred to or received from the interface component. The light applicator can be configured to apply antimicrobial light to airborne pathogens transferred to and received from the interface component. The interface component can include various components, such as an endotracheal tube or a breathing mask. The light applicator can include a first port and a second port and be configured to apply antimicrobial light to airborne pathogens transferred between the first port and the second port. The light applicator can apply antimicrobial light that includes one or more of various wavelengths, such as an ultraviolet wavelength.

In another aspect, a method for treating toenail fungus is provided. The method includes identifying at least one toenail having a toenail fungus, engaging a patient's foot having said at least one toenail with a holding fixture, determining a scan pattern for light so as to cover at least one region of said at least one toenail, and scanning light in the scan pattern so as to irradiate said at least one region with a wavelength corresponding to an absorption spectrum of the toenail fungus.

In many embodiments, the holding fixture includes one or more features. For example, the holding fixture can include at least one toe separation feature so that the step of engaging a patient's foot with the holding fixture includes positioning at least two of the patient's toes on opposite sides of the at least one toe separation feature. The holding fixture can include a heel support that engages the patient's heel and a strap to secure the patient's foot to the holding fixture.

In many embodiments, the method for treating toenail fungus further includes capturing a first image that includes the at least one toenail, and processing the first image so as to identify the at least one region. The method can further include storing the first image (*e*.*g*., in computer memory, etc.). The first image can be processed using edge detection to identify at least one toe of the patient and/or to identify at least one toenail of the patient. The first image can also be displayed and one or more user inputs accepted to identify the at least one region. In many embodiments, a composite image is displayed that includes a first scaled image of a first toenail and a second scaled image of a second toenail, the first and second images being scaled so as to substantially match a displayed size of the first and second images. An image can be captured during a treatment session and processed and/or displayed to provide feedback to a system operator during the treatment session.

In many embodiments, a displayed image includes a depiction of at least a portion of a scan pattern. In many embodiments, a scan pattern can be modified and the modified scan pattern can be displayed.

In many embodiments, the method for treating toenail fungus can include a subsequent treatment session. For example, the method can further include re-engaging the patient's foot with the holding fixture during a second treatment session, capturing a second image that includes said at least one toenail, displaying the second image, and displaying the first image.

In another aspect, a method for treating a patient with a licensed treatment system is provided. The method includes receiving a payment for a patient treatment; in response to a license fee payment being made for the use of a licensed treatment system, receiving a communication that is used to enable the system; and providing the patient treatment with the system. In many embodiments, the patient treatment includes treating a toenail fungus by irradiating a toenail of a patient with light comprising a wavelength corresponding to an absorption spectrum of the toenail fungus. In many embodiments, an automated scan pattern is used to irradiate the toenail.

In another aspect, a method for interacting with a user of a toenail fungus treatment system is provided. The treatment system includes a display and an input device. The method includes displaying an image on the display comprising at least a portion of a toenail of a patient and a representation of at least a portion of a scan pattern for irradiating at least one toenail of the patient with light comprising a wavelength corresponding to an absorption spectrum of the toenail fungus, and receiving a user input via the input device to accept the scan pattern or modify the scan pattern. In many embodiments, the method further includes at least one of providing the user access to existing patient information, receiving new patient information from the user, providing the user access to patient treatment history information, or receiving user input to manually designate at least a portion of the scan pattern.

In another aspect, a method of interacting with a user of a toenail fungus treatment system is provided. The treatment system includes a display and an input device. The method includes displaying an image on the display comprising at least a portion of a toenail of a patient and a representation of at least a portion of a scan pattern for irradiating at least one toenail of the patient with light comprising a wavelength corresponding to an absorption spectrum of the toenail fungus, and displaying a representation of the scan pattern on the display to indicate an accomplished portion of the scan pattern. In many embodiments, a series of representations of the scan pattern are displayed so as to indicate progression of the treatment.

In another aspect, an automated toenail fungus treatment system is provided. The system includes a light scanner operable to irradiate a patient's toenail with a penetrating antimicrobial light according to a scan pattern, a light source coupled with the light scanner to supply the penetrating antimicrobial light to the light scanner, and a controller coupled with the light source and the light scanner, the controller comprising a processor and a tangible medium comprising instructions that when executed cause the processor to control the operation of the light source and the light scanner.

In many embodiments, an automated toenail fungus treatment system includes at least one additional component and/or additional functionality. For example, an automated treatment system can include an imaging device coupled with the light scanner and operable to capture an image of the patient's toenail. An automated treatment system can include a display coupled with the controller. The instructions, when executed, can further cause the processor to display an image of the patient's toenail on the display. The instructions, when executed, can further cause the processor to display a representation of at least a portion of the scan pattern on the display. An automated treatment system can include an input device. The instructions, when executed, can cause the processor to modify the scan pattern in response to one or more user inputs. An automated treatment system can further include a holding fixture configured to receive and position a patient's foot so as to position the patient's toenails relative to the light scanner. An automated treatment system can further include a manual light applicator coupled with the light source.

For a fuller understanding of the nature and advantages of the present invention, reference should be made to the ensuing detailed description and accompanying drawings. Other aspects, objects and advantages of the invention will be apparent from the drawings and the detailed description that follows.

For a fuller understanding of the nature and advantages of the present invention, reference should be made to the ensuing detailed description and accompanying drawings.

### BRIEF DESCRIPTION OF THE DRAWINGS

**FIG. 1** is a flow chart of a method for determining an antimicrobial light treatment for a pathogen, in accordance with many embodiments.
**FIG. 2** is a block representation of components for an antimicrobial light treatment system, in accordance with many embodiments.
**FIG. 3** is a graph representing a Monte Carlo simulation of the penetration of antimicrobial treatment light into a toenail, in accordance with many embodiments.
**FIG. 4A** is a representation of a burst mode for an antimicrobial laser treatment, in accordance with many embodiments.
**FIG. 4B** is a representation of a burst mode that can be used for an automated antimicrobial laser treatment, in accordance with many embodiments.
**FIG. 5** is a sectional representation of a toe.
**FIG. 6** illustrates the treatment of toe fungus via the application of antimicrobial light, in accordance with many embodiments.
**FIG. 7** illustrates a plantar wart and the treatment of a plantar wart via the application of antimicrobial light, in accordance with many embodiments.
**FIG. 8A** is a schematic representation of components for an antimicrobial light system that includes a perimeter light applicator, in accordance with many embodiments.
**FIG. 8B** is a schematic representation of components for an antimicrobial light system that includes a wand light applicator, in accordance with many embodiments.
**FIG. 8C** is a schematic representation of components for an antimicrobial light system that includes a light applicator having an array of light emitters, in accordance with many embodiments.
**FIG. 9** is a sectional representation of a tooth having a periapical abscess.
**FIG. 10** is a sectional representation of a root-canalled tooth, illustrating the application of an antimicrobial light treatment, in accordance with many embodiments.
**FIG. 11** is a sectional representation of a human auditory system, illustrating the application of an antimicrobial light treatment from within the ear canal, in accordance with many embodiments.
**FIG. 12** is a sectional representation of internal cavities within a human head and neck, illustrating the application of antimicrobial light treatments from within the sinus cavity and to the vocal cords, in accordance with many embodiments.
**FIG. 13** is a sectional representation of a lower portion of a female patient, illustrating the application of an antimicrobial light treatment to the cervix, in accordance with many embodiments.
**FIG. 14** illustrates the treatment of osteomyelitis within the foot of a patient via the external application of an antimicrobial light treatment from multiple directions, in accordance with many embodiments.
**FIG. 15** is a representation of the digestive system of a patient, illustrating the application of antimicrobial light treatments from within the stomach and from within the large intestine, in accordance with many embodiments.
**FIG. 16** is a sectional representation of a lower portion of a male patient, illustrating the application of an antimicrobial light treatment from within the urinary tract, in accordance with many embodiments.
**FIG. 17** is a representation of the circulatory system of a patient, illustrating the application of an antimicrobial light treatment from within a blood vessel, in accordance with many embodiments.
**FIG. 18A** is a sectional representation of the pulmonary system of a patient, illustrating the application antimicrobial light to air transported within the trachea, in accordance with many embodiments.
**FIG. 18B** is a view along a patient's trachea showing a simplified representation of an antimicrobial light applicator positioned within the trachea, in accordance with many embodiments.
**FIG. 19** is a representation of an antimicrobial light applicator coupled with an endotracheal tube inserted within a patient, illustrating the application of antimicrobial light to treat pathogens entering and/or leaving a patient's respiratory system, in accordance with many embodiments.
**FIG. 20A** is a schematic diagram of a patient foot engaged with a holding fixture configured to receive and position the patient's foot so as to position the patient's toenails relative to a light scanner, in accordance with many embodiments.
**FIG. 20B** is a schematic diagram of a patient foot engaged with a holding fixture having toe straps used to position the patient's toenails relative to a light scanner, in accordance with many embodiments.
**FIG. 21A** is a schematic diagram illustrating a patient's toes separated by toe separation features of the holding fixture of **FIG. 20** and corresponding to a field-of-view of the light scanner of **FIG. 20****,** in accordance with many embodiments.
**FIG. 21B** illustrates the display of an image of a patient's toenail, the toenail image being scaled and reoriented, in accordance with many embodiments.
**FIG. 21C** illustrates the display of a composite image showing five images of a patient's toenails, the toenail images being reoriented and scaled to approximately the same size, in accordance with many embodiments.
**FIG. 22A** illustrates the display of an image of a patient's toenail showing a representation of a treatment scan pattern for the toenail, in accordance with many embodiments.
**FIG. 22B** illustrates a scan pattern in which a sequence of pulses are spaced apart to limit thermal accumulation, in accordance with many embodiments.
**FIG. 23** is a flow chart of a method for treating toenail fungus, in accordance with many embodiments.
**FIG. 24** is a flow chart of a method for treating a patient with a licensed treatment system, in accordance with many embodiments.
**FIG. 25** is a simplified block diagram of an automated antimicrobial light treatment system, in accordance with many embodiments.
**FIG. 26** is a simplified block diagram of functions that can be accessed through a user interface of an automated antimicrobial light treatment system, in accordance with many embodiments.
**FIG. 27** shows a graphical user interface screen for accessing existing patient information via a touch-screen display of a toenail fungus treatment system, in accordance with many embodiments.
**FIG. 28** shows a graphical user interface screen for setting system preferences via a touch-screen display of a toenail fungus treatment system, in accordance with many embodiments.
**FIG. 29** shows a graphical user interface screen displaying images of a patient's toenails from one or more treatment sessions, in accordance with many embodiments.
**FIG. 30** shows a graphical user interface screen for selecting a patient's toenail for treatment via a touch-screen display of a toenail fungus treatment system, in accordance with many embodiments.
**FIG. 31** shows a graphical user interface screen displaying a scan pattern for treating a patient's toenail via a toenail fungus treatment system, in accordance with many embodiments.

### DETAILED DESCRIPTION

In the following description, various embodiments of the present disclosure will be described. For purposes of explanation, specific configurations and details are set forth in order to provide a thorough understanding of the embodiments. However, it will also be apparent to one skilled in the art that the present invention may be practiced without the specific details. Furthermore, well-known features may be omitted or simplified in order not to obscure the embodiment being described.

Embodiments disclosed herein are generally directed to the treatment of infectious diseases by using antimicrobial light to combat the responsible pathogen(s). The antimicrobial light used can be selected and applied so that it is at least partially absorbed by the responsible pathogen(s), which weakens, sterilizes, and/or kills the pathogen(s). In some embodiments, the antimicrobial light is selected to optimize the ratio of harm to the pathogen to the harm to the host cause by the application of the antimicrobial light. In some embodiments, the antimicrobial light is selected and/or applied such that it at least partially penetrates through host cells prior to reaching and being absorbed by the pathogen(s).

Antimicrobial light, in the form of short wavelength ultraviolet light (UVC) has been used in a sterilization method known as ultraviolet germicidal irradiation (UVGI). UVC is harmful to some forms of life. At a wavelength of 254 nm, ultraviolet light (UV) will break down the molecular bonds within the DNA of some pathogens, thereby destroying them, rendering them harmless, and/or prohibiting their growth and/or reproduction. Germicidal UV can be delivered by a mercury-vapor lamp (mercury vapor emits light at 254 nm).

The effectiveness of UVGI is dependent on line-of-sight exposure of the pathogen to the UVC light. Obstacles serve to shelter the pathogen(s) from the UVC light, thereby degrading the effectiveness of UVGI.

There are a number of potential dangers associated with the use of UVC light. In addition to being harmful to some pathogens, UVC is harmful to humans and other forms of life. Exposure to UVC can cause sunburn and, in some cases, cancers, such as skin cancer. Exposure of the eyes to UVC can produce painful inflammation of the cornea and temporary or permanent vision impairment or blindness. UVC can damage the retina of the eye. UVC can also result in the production of ozone, which can be harmful to a person's health. Most UVGI systems are designed to limit or prevent exposure to UVC light.

In embodiments, a number of approaches are used for avoiding problems associated with the application of UVC light. In one approach, non-UVC wavelengths are used. In another approach, wavelengths are selected that can penetrate into host tissue, and thereby reach pathogen(s) disposed within or behind the host tissue. In another approach, UV light is used to kill airborne pathogens from within a host airway. The application of the UV light can be accomplished so as to minimize damaging host tissue by minimizing the exposure to the UV light and/or by applying UV light to areas of the host that are more resistant to being damaged by exposure to UV light.

An advantage to using an antimicrobial laser or other antimicrobial light is that antibiotic resistant diseases can be treated. Pathogens for a disease can be destroyed with antimicrobial light (*e*.*g*., laser pulses, etc.), which may be transmitted through a host (*e*.*g*., through host cells such as the nail plate on a patient's toe) without damage to the host.

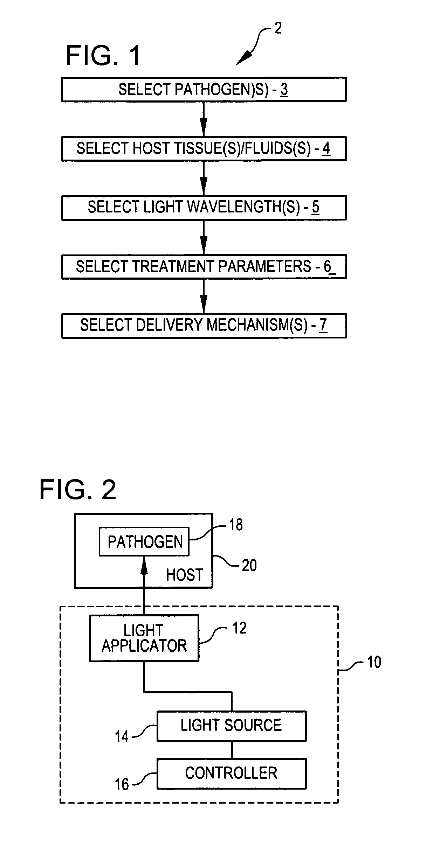

**FIG. 1** is a flow chart showing a method **2** for determining a treatment for combating a pathogen disposed within a host in accordance with an embodiment. The method includes the selection of pathogen(s) (action **3**) to be targeted, the selection of host tissue(s) and/or host fluid(s) (action **4**) to be irradiated during the treatment, the selection of light wavelength(s) (action **5**) for the antimicrobial light, the selection of treatment parameters (*e*.*g*., energy level, pulse shape, pulse duration, pulse frequency, burst frequency, etc.) (action **6**) for the application of the antimicrobial light, and the selection of a delivery mechanism(s) (action **7**) for the application of the antimicrobial light.

**FIG. 2** is a block representation of components for an antimicrobial light treatment system **10** in accordance with an embodiment. The system includes a light applicator **12,** a light source **14,** and a controller **16.** The system **10** can be used to apply antimicrobial light to a pathogen(s) **18** disposed on and/or within a host **20.** For example, the pathogen(s) **18** can be a toenail fungus located on a patient's toe nail bed (disposed underneath the patient's toe nail plate). The light applicator **12** can be used to target the pathogen(s) **18** that the treatment is designed to destroy. The host **20** is the tissue or other environment in which the target pathogen(s) **18** resides. The light source **14** is connected to the controller **16.** The controller **16** can provide control signals and/or instructions to the light source **14,** including light parameters, and can be used to control the light supplied to the light applicator **12.** The controller **16** can be a standard control (*e*.*g*., a device or mechanism used to regulate or guide the operation of a machine, apparatus, or system), a microcomputer, or any other device that can execute computer-executable instructions, such as program modules. Generally, program modules include routines, programs, objects, components, data structures and the like that perform particular tasks or implement particular abstract data types. A programmer of ordinary skill in the art can program or configure the controller **16** to perform the functions described herein.

According to the invention, the light source **14** is a laser. In an embodiment, the laser is a Nd:YAG laser. Laser devices for use in protocols established by embodiments described herein include, but are not limited to, a pulsed free-running Nd:YAG fiber delivered laser; a pulsed free-running Nd:YAG free-beam delivered laser with automated scanning interface; a Q-switched Nd:YAG fiber delivered laser; a Q-switched Nd:YAG free-beam delivered laser with automated scanning interface; a diode 1064 nm pulsed fiber delivered laser, a diode UV free-beam delivered laser; and a very large high-powered confocal beam Nd:YAG (free-running or Q-switched) delivered via gel-interface concave fiber/lens directly to tissue for targeted depth of penetration into tissue.

The light applicator **12** is connected to the light source **14.** The light applicator **12** is utilized for delivery of the light generated by the light source **14** to the target pathogen(s), and may be, for example, an optical fiber, a wave guide, an articulated arm, a lens system, or another suitable structure. As further described below, the light applicator **12** can be integrated with existing endoscope configurations, such as, but not limited to, endoscopes used to view within the gastrointestinal tract during esophagogastroduodenoscopy, colonoscopy, or proctosigmoidoscopy; within the respiratory tract during rhinoscopy or bronchoscopy; within the urinary tract during cystocopy; within the female reproductive system during colposcopy, hysteroscopy, or falloscopy; within normally closed body cavities (*e*.*g*., through a small incision) during laparoscopy, anthroscopy, thorascopy, or mediatinoscopy; and within the uterus during amnioscopy or fetoscopy.

In addition to endoscopic configurations employing wire and/or optical cables, the light applicator **12** can be integrated with wireless endoscopic configurations, such as a wireless pill endoscopic camera. In embodiments, the position and/or orientation of a pill-camera light applicator within a patient can be controlled through the selective application of a magnetic field. Power can be supplied to the pill-camera light applicator via inductive charging via an external coil, which may be located in a vest worn by the patient. Images obtained by the pill-camera light applicator can be wireless transmitted to an external antenna for external viewing and/or storage in a memory device. The images can also be stored within a memory device resident on the pill-camera light applicator. A pill-camera light applicator can also include one or more mechanisms to vary the orientation of the camera and/or the light applicator. Antimicrobial light can be selectively applied by varying the position and/or orientation of the pill-camera light applicator via the applied magnetic field, and/or by varying the orientation of the light applicator via the one or more orientation mechanisms (*e*.*g*., a rotational mechanism, a pivot mechanism, etc.).

To determine a treatment regime, light wavelengths are determined that correspond to an absorption spectrum of the targeted pathogen(s). To determine the appropriate wavelength(s), the absorption spectra for the target pathogen (*e*.*g*., a toenail fungus) are obtained. This may be performed, for example, by performing spectroscopy on the target to identify absorption of light photons by the target pathogen(s) over a spectrum of different wavelengths. When obtaining the absorption spectra, the target pathogen is characterized with spectroscopy to identify light-absorbing molecules or chemical bonds with absorption peak wavelengths (targeting wavelengths) that are unique to that pathogen. There may be more than a single targeting wavelength.

For example, to identify an appropriate wavelength of light for killing toenail fungus pathogens, spectral analysis was conducted on a culture of toenail fungus. Through analysis, it was discovered that there were several pathogens in the toenail fungus. In an embodiment, the laser treatment wavelength is chosen such as to maximize the collective absorptions of the pathogens within a mixture of target pathogens. Alternatively, different treatment procedures can be developed for each pathogen or for a selected group of pathogens within the mixture of target pathogens. In the present example involving toenail fungus, the spectral analysis identified four distinct wavelengths that have high differential absorption for most if not all of the target pathogens.

Although any of these four wavelengths may be used, an analysis was done to find a suitable wavelength. Of the four wavelengths, the first was in the ultraviolet spectrum. As is known, ultraviolet spectrum wavelengths potentially cause cancer in tissue. Thus, that wavelength was eliminated for this reason, but could be pursued under controlled conditions. The remaining three wavelengths were in the infrared (IR) spectrum. The first of these three, approximately 9 microns, was rejected because, although commercially available lasers are capable of producing laser pulses at that wavelength, the laser pulses do not have sufficient penetration depth to penetrate a toe nail plate. The second of the two IR wavelengths was approximately 4 microns, which is not producible by commercially known laser systems. Therefore, that wavelength was also eliminated. The last wavelength, approximately 1064 nanometers, is readily produced by an Nd:YAG laser source. Therefore, that wavelength was selected.

Formulating a treatment regime can also involve obtaining/determining an absorption spectra for the host. The host tissue can be characterized with spectroscopy to determine the major contributors to the absorption of light. As an example, in U.S. Patent No. 7,090,497, the entire disclosure of which is incorporated by reference, water, hemoglobin, and melanin were analyzed to identify absorption spectra. In addition to these contributors, for toenail fungus, hydroxylapatite (a mineral in bone, nails and teeth) was analyzed. In accordance with that evaluation, the absorption spectra showed that there is a preferred window of wavelengths that are at least partially non-absorbing for water, hemoglobin, hydroxylapatite, and melanin corresponding approximately to light having wavelengths between 550 and 1,800 nm. The wavelength of 1064 nm, set forth above, falls in this range, and thus that wavelength was selected as being both toxic to the target and transmissible through the host. Other wavelengths may be selected, but preferably a wavelength or range of wavelengths for use in the methods herein will have a sufficiently high therapeutic ratio. By "therapeutic ratio," we mean a ratio of the minimum laser fluence damaging to the host divided by the minimum laser fluence that destroys the target. Preferably, the light absorption by the pathogen at the targeting wavelength is considerably more efficient than the absorption by the surrounding host at the targeting wavelength. As such, a high number is typically a favorable therapeutic ratio, and likely lethal to the target, with relatively little effect to the host.

A toxic dose for the host can be determined. This evaluation involves a determination of a range of wavelengths and scattering coefficients that are acceptable to the host (*e*.*g*., do not cause excessive damage to or pain for the host) and provide adequate tissue penetration. The absorption or transmission spectra of the host found above are evaluated, as well as damage thresholds and responses to treatment of the host. This process can involve Monte Carlo simulations to evaluate the scattering and absorption within the host of the light photons from the laser pulses. These simulations are helpful for determining both the light and the heat distribution caused by a light source, which in turn may indicate potential pain or damage. Monte Carlo simulations are also helpful to determine the depth of kill for specific pathogens, host tissues, and light sources. Monte Carlo simulations are known and are commercially available. An example is described in "A Monte Carlo Model of Light Propagation in Tissue," SPIE Instuture Series Vol. IS 5 (S. A. Prahl et al., 1989). Utilizing a Monte Carlo simulation, a determination can be made whether the penetration is sufficient to reach the target pathogen(s), and whether the laser fluence at that penetration level is sufficient to combat the targeted pathogen(s). If not, a higher energy level may be needed for the pulse, more pulses may be needed, or the pulse may need to be concentrated into a smaller area, permitting greater penetration. A smaller spot size, however, may not cover the entire pathogen, and thus a distribution of additional pulses may be needed, or higher pulse energy may be required.

In general, pathogens in toenail fungus are small, and thus a short pulse duration was selected for an initial test. By "pulse duration," we mean the temporal length of the pulse, as well as the speed of the rise and decay of the energy for the pulse. For example, the pulse may be all power on and all power off or it may gradually rise in power and gradually decrease or decay in power. In addition, the pulse may gradually rise in power and then dramatically drop or dramatically rise in power and gradually decay. Any combination may be used. To determine the duration and the rise and decay of the pulse duration, the size and shape of the target is taken into consideration. Generally, a smaller target requires less pulse duration and faster rise and decay. Thermal relaxation of the target is also taken into account. For example, when a rapid rise or shorter pulse is utilized, the temperature of a target may increase faster than it dissipates, meaning that the pulse may require less energy to effectively combat the target pathogen(s). In an embodiment, an 80 microsecond duration pulse was selected for use against the toe nail fungus, although other pulse durations may be used.

Pulse energy can also be determined for a treatment. This evaluation involves determining the energy needed for destruction of the target pathogen. Typically, there is an energy threshold at which destruction of the target pathogen occurs, and this threshold can be determined empirically and/or clinically. As part of this evaluation, however, the effect of the energy level on the host is taken into account. For example, a threshold that permits destruction of the target pathogen may result in damage or intolerable pain to the host. Preferably, pulse energy is selected that is above the energy threshold, is as high as possible, but does not damage or harm the host. Using the Nd:YAG laser source, pulse energy of 200mJ was determined to be acceptable to the host toenail, and destructive to the toenail fungus, so this pulse energy was selected, although other pulse energy levels may be used.

The depth of the target pathogen(s) in the host can be considered in determining the pulse energy that is needed. According to the Beer-Lambert law, the intensity of an electromagnetic wave inside a material falls off exponentially from the surface. Thus, in general, with all other parameters remaining constant, the greater the desired depth of irradiation, higher pulse energy is required to achieve the intensity at the target pathogen(s) necessary for destruction. However, the surface irradiation must also be kept below the damage threshold for the host. For toenail fungus, an assumption was made that the nail plate is 0.4 millimeters thick, and that fungus resides under the nail plate N. In an embodiment, an assumption was made that the fungus is 0.1 millimeters thick.

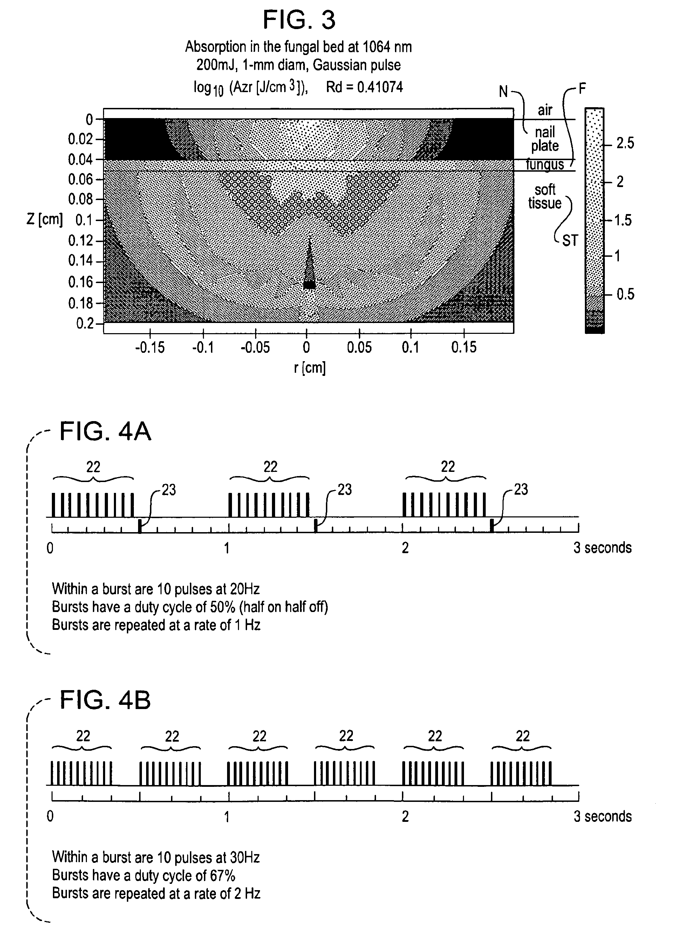

A Monte Carlo simulation was conducted of a toe having a 0.4 millimeter thick nail plate, a 0.1 millimeter thick fungal mat under the nail, and an infinite layer of soft tissue underneath the fungal mat. The simulation assumed that the fungus is located under the nail plate, for example in the nail bed, but as described above, the fungus may be in the nail plate or on top of the nail plate. The results are shown in **FIG. 3****.** As can be seen, there is a large amount of photon absorption in the fungus **(F)** (as indicated by the relatively lighter color associated with the region of the fungus as compared to adjacent areas), and apparent refraction by the nail plate **(N)** and the soft tissue **(ST)** (as indicated by the dispersion pattern that results from the centrally located energy pulse). From the simulation, it appears that some of the photons penetrate the nail plate **(N),** and then are absorbed by the fungus **(F)** trapped between the nail plate **(N)** and the soft tissue **(ST)**, either immediately being absorbed by the fungus, or refracting between the nail plate and the soft tissue until absorbed by the fungus.

Evaluation of light distribution in the host via the Monte Carlo simulation resulted in selection of a spot size of slightly greater than 1 millimeter. This spot size provides sufficient light distribution to below the nail plate. However, because a toenail fungus colony typically has a much larger size than this dimension, a decision was made to irradiate several spots in a systematic fashion to ensure treating of the entire infected area.

A treatment pattern for the pulses was then determined, including a burst mode and a repetition rate. A burst is a series of pulses, which may or may not be followed by a gap in which there are no pulses. The repetition rate of the pulses in a burst is an interval between pulses within the burst (*e*.*g*., 20 Hz). Bursts may be repeated, and as such there may be a repetition rate for the bursts. In such case, the burst repetition rate represents the duty cycle of the bursts (*i*.*e*., how often a burst is repeated). For example, after a burst having X number of pulses, another burst having the same or different number of pulses can follow immediately or after a delay period.

For example, as shown in **FIG. 4A****,** a burst **22** may last for 1/2 second and have 10 pulses (*i*.*e*., pulses repeated at 20 Hz), with the duty cycle being one half on, and one half off for the bursts. In this example, the repetition rate of the pulses is 20 Hz, and the repetition rate of the bursts is 1 Hz. In many embodiments, the power used to apply the sequence of bursts shown in **FIG. 4A** is approximately 4 watts. At the end of the burst **22,** an indication **23** (*e*.*g*., a light signal and/or an audible tone) can be optionally used to signal the completion of the burst **22** such that the light applicator **12** can be repositioned for the application of the next burst **22** at another location. The resulting sequence of indications **23** may serve to instill in the operator of the light applicator **12** a sense of timing of the sequence of bursts and thereby allow the operator to better move the light applicator **12** in phase with the sequence of bursts. While the use of the indications 23 may be beneficial, it is not required.

As can be understood from the discussion of above, increasing the number of pulses in a burst, or the number of bursts, may increase the depth and width of a treatment. However, increasing the number of pulses may also result in damage or harm to the host by excess heating. Cycling of the laser energy (*i*.*e*., the duty cycle) on and off between bursts can help to limit the thermal accumulation in the host, yet still provides thermal accumulation in the target. Additionally, the use of pulsed application allows for higher peak energy emitted, which results in higher peak energy level transmitted to the pathogen while limiting of thermal accumulation within the host tissue.

Additional bursts may be applied at the same place or to a different location so as to address a separate portion of the target pathogen(s). The additional bursts can be limited due to host damage or patient tolerance. Operator skill may also determine the number of bursts that can be applied. That is, an operator's skill may limit how fast a burst operation may be repeated, or how accurately multiple bursts may be applied to different locations on a patient. To this end, in some applications, a computer or other controller can be utilized to provide multiple bursts directed to different locations on a patient, for example in a raster pattern directed by a controller such as a computer.

When an operator manually scans the light applicator **12**, it may be beneficial to employ a scan pattern so as to generate a more uniform coverage for the light application. Such a scan pattern can involve, for example, one or more raster patterns. Where two or more raster patterns are used, it may be beneficial to vary the orientation of two or more of the raster patterns and thereby ensure more complete coverage of the area being treated. For example, a first raster pattern in which the light applicator is moved across the nail (*e*.*g*., from left to right and/or from right to left to apply a sequence of bursts to adjacent locations so as to sequentially treat rows of locations) can be used, followed by a second raster pattern in which the light applicator is moved in another direction (*e*.*g*., from top to bottom and/or from bottom to top to apply a sequence of bursts to adjacent locations so as to sequentially treat columns of locations).

The sequence of bursts can be tailored to suit the method of application. For example, the sequence of bursts illustrated in **FIG. 4A** can be used, for example, with a manually positioned light applicator. When an automated method of application is used, such as an automated light scanning system, the sequence of bursts can be tailored to suit the positional accuracy and movement speed of the system employed. For example, **FIG. 4B** shows a burst mode that can be used in an automated antimicrobial light treatment. In **FIG. 4B****,** each of the bursts **22** include 10 light pulses applied over one-third second durations (*i*.*e*., pulses repeated at 30 Hz) instead of the one-half second durations of **FIG. 4A****.** This reduction in burst duration may be acceptable from a patient comfort standpoint due to the increased accuracy of an automated light scanning system as compared manual scanning. Additionally, an automated light scanning system can be used to apply a more uniform intensity of light over a treatment location, thereby requiring less time between pulses to avoid excessive accumulation of thermal energy. Each of the bursts **22** are separated by only one-sixth of a second. Such a reduction in the separation time between bursts may be achievable due to the greater speed and/or positional control of an automated light scanning system. The intensity of each pulse in each burst **22** can also be tailored to suit the method of application. In many embodiments, the power used to apply the sequence of bursts shown in **FIG. 4B** is approximately 6 watts.

By increasing the number of pulses, the amount of energy for each pulse at a given depth within the host may be decreased. However, the use of multiple pulses may result in thermal heating of the host, which may cause pain or damage to the host. Thus, an evaluation can be made whether the increased number of pulses is damaging to the host. This can involve testing to determine patient tolerance.

Combating pathogens with antimicrobial light can be accomplished in a variety of ways. For example, antimicrobial light can be applied to combat pathogens disposed on and/or within host tissues. Antimicrobial light can be applied to combat pathogens disposed within bodily fluids, such as blood. Antimicrobial light can be applied to combat airborne pathogens from within host airways. As will be discussed in more detail below, these approaches can be used to combat pathogens located in a multitude of locations on and/or within a patient.

### Combating Surface Pathogens and/or Pathogens in Tissue

Antimicrobial light can be used to combat surface pathogens and/or pathogens in tissue in a wide variety of medical areas. As non-exclusive examples, such medical areas include: podiatry/dermatology; dentistry; ear, nose, and throat; obstetrics/gynecology; osteopathy; gastroenterology; and urology.

A range of medical conditions can be treated. These conditions include warts (such as common warts and plantar warts), diabetic ulcers, cold sores, acne, and wounds (*e*.*g*., burns, surgical wounds, trauma, and ulcers). With at least some of these treatments (*e*.*g*., diabetic ulcers and wounds), antimicrobial light can be used to disinfect the host tissue, thereby helping to establish better healing conditions. Antimicrobial light treatment can be used alone, or in conjunction with other treatments (*e*.*g*., drug therapy, light stimulation therapy, etc.).

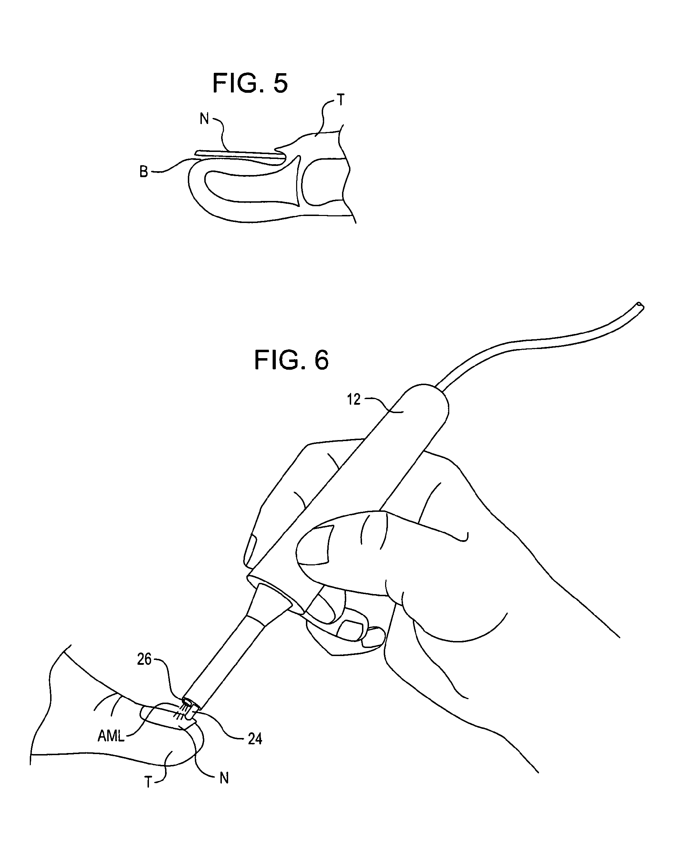

Antimicrobial light treatments in podiatry and/or dermatology can be externally applied. In one example (discussed above), a treatment for toenail fungus can be externally applied. Toenail fungus may reside in the toenail, under the toenail and/or on the top surface of the toenail. For example, as shown in **FIG. 5****,** a toe **(T)** has a nail plate **(N)** with a nail bed **(B)** underneath. The nail bed **(B)** is typically the adherent connective tissue that underlies the nail plate **(N).** The nail plate **(N)** is typically hard and translucent, and is formed of keratin.

Toenail fungus pathogen(s), such as the pathogen(s) for onychomycosis, typically reside in the nail plate **(N),** the nail bed **(B),** just under the nail bed, between the nail bed and nail plate, or on top of the nail plate. One issue in the treatment of toenail fungus pathogen(s) is that a treatment, if topical, must penetrate the nail plate **(N)** without causing harm to the nail plate. Accordingly, embodiments described herein seek to eradicate such toenail pathogens in the nail plate and/or under the nail plate without harming the nail or its growth. Such eradication can be provided by antimicrobial light, for example antimicrobial laser pulses or sequences of laser pulses.

Testing on patients (for toenail fungus treatment) revealed that 10 pulses at 20 Hertz appear to be a threshold at which there is little or no experience of pain for most patients. Thus, this burst mode was selected. A laser applicator, such as the light applicator **12** shown in **FIG. 6****,** was used that included a spacer **24** for properly spacing a fiber tip from the nail plate **(N).** The spacer **24** is positioned so that it touches the nail plate **(N),** thus properly spacing a fiber tip **26** for the light applicator the appropriate amount from the nail plate. If a patient experienced pain during the application of the antimicrobial light **(AML),** the distance of the light applicator from the nail plate was increased by lifting the spacer **24** away from the nail plate **(N),** creating a larger spot size.

The antimicrobial light treatment for toenail fungus described above has shown to be safe and effective in a first formal clinical study. In a study of 16 patients, a 90 day longitudinal randomized controlled study was conducted at a private podiatric practice with a single treatment for toenail fungus. The study resulted in 88% of toenail fungus patients, treated one time, gaining an average of 3.9 mm clear nail growth in 90 days with 100% of patients having no side effects. This is better efficacy and safety than the current standard of care, with no patient compliance requirements.

In the study, a single 1mm spot was irradiated on an infected toenail holding a 0.22 na fiber tip 3 mm from the nail surface (for example, using the spacer **24** described above). The Nd:YAG laser delivered a 1/2 second pulse train of 10 200 mJ, 80 microsecond duration pulses at 20 Hz, and repeated at one second intervals. During the 1/2 second period in which the pulse train was off, the fiber tip was moved to a new location. Spots were irradiated sequentially in a systematic fashion across the surface of the toenail to ensure treatment of the entire nail including 2 mm of proximal nail fold, 2 mm of each lateral nail fold and 2 mm of toe skin in front of/under a distal edge (free edge) of the nail. If the subject could not tolerate a 10 pulse train, then the number of pulses was decreased, or the tip was moved away from the toenail, until tolerated.

A scanner can be used during the application of antimicrobial light. The scanner can be used to project a spot pattern on the toenail, which can be used as reference marks by a technician during manual application of the antimicrobial light. Alternatively, the scanner can be connected to a controller, such as the controller **16,** which automatically applies the antimicrobial light in a raster pattern, eliminating any opportunity for operator error.

Nineteen pilot patients with toenail fungus were treated on one toe and followed for three months. One great toe was treated on each patient and the other was left untreated to serve as a control. Treatments were randomized to right or left toes. Patients were evaluated for culture results, notch measurements (nail plate growth), and lesion measurements (nail bed clearing) pre and post treatment at one week, one month, two months, and three months (notch and lesion only). Notch and lesion analysis used data for the 16 subjects who completed all follow-up evaluations. The distance from the proximal fold to the notch or to the lesion increases as the nail plate grows or the nail bed clears, respectively.

Notch (nail plate growth) measurements increased in a similar fashion with no difference between treated and untreated toes. There was a highly significant difference (p<0.001) between treated and untreated toes for the lesion (nail bed clearing) measurement. For treated toes, lesion average values went from 4.0 mm at baseline to 7.7 mm at 90 days, while the untreated toes went from 5.3 mm at baseline to 5.6 mm at 90 days. Fourteen out of 16 (87.5%) treated toes improved. Improvement ranged from 1.07 mm to 10.41 mm in 90 days following a single treatment. For the 14 toes that responded to therapy the average improvement was 3.9 mm, or 1.3 mm per month.

In conclusion, there was no discrimination between treatment and no treatment for notch (nail plate growth) measurements; however there were highly significant treatment differences for lesion (nail bed clearing) measurements.

Plantar warts are another example of a medical condition that may be treatable by the application of penetrating antimicrobial light. A plantar wart is caused by a virus that lives in the host tissue to an unknown depth. A plantar wart does not appear to be particularly responsive to just surface radiation. The virus may be like other viruses, such as herpes, that follow a nerve root or that have a viral reservoir somewhere deeper in the host tissue. If a plantar wart(s) is surgically removed, to the extent that it is visible, from a patient, in a few weeks time the plantar wart(s) may return.

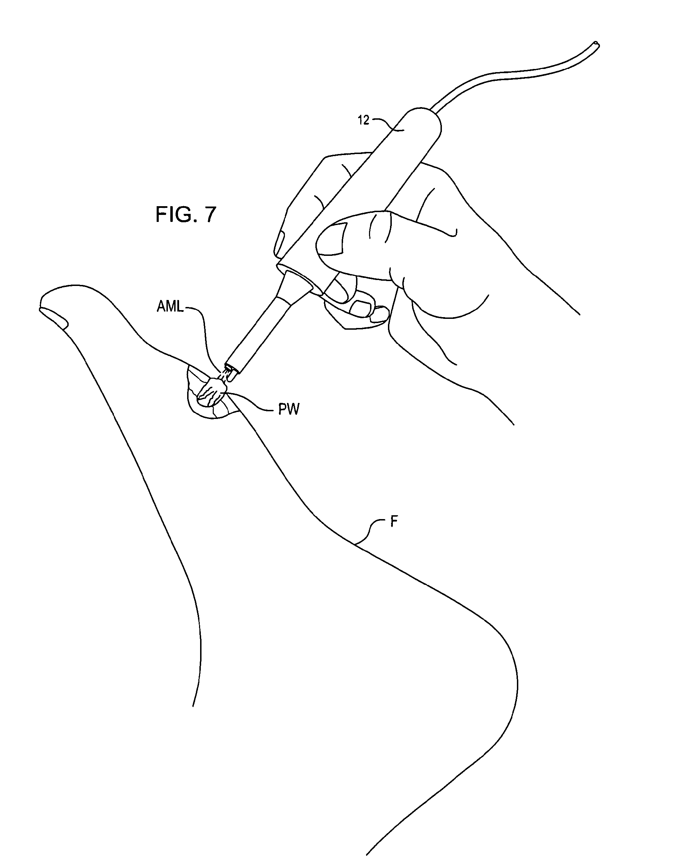

For some plantar warts that are located within an effective penetrating distance from the bottom of a patient's foot, it may be possible to treat the wart as illustrated in **FIG. 7****.** As illustrated, a plantar wart (**PW**) may have a body that is largely disposed within a patient's foot **(F),** with a small portion being visible from the exterior of the patient's foot **(F).** A light applicator **12** can be used to administer penetrating antimicrobial light **(AML)** to the plantar wart **(PW).** The parameters of the treatment (*e*.*g*., wavelength, pulse rate, pulse duration, pulse energy, burst mode, surface irradiance, spot size, scanning, etc.) can be determined using the approaches discussed above with regard to the treatment of toenail fungus.

Based upon the above discussed treatment that was developed for toe fungus, it is believed that antimicrobial light wavelengths and treatment parameters can be developed for the treatment of many surface pathogens and/or pathogens located within host tissue. For example, it is believed that various host tissues may be at least partially non-absorbing of light having certain wavelengths, such as between 550 and 1,800 nm. It is also believed that a light wavelength(s) can be identified using the above discussed approach that is both at least partially transmissible through a host tissue while being absorbed by, and toxic to, a selected pathogen.

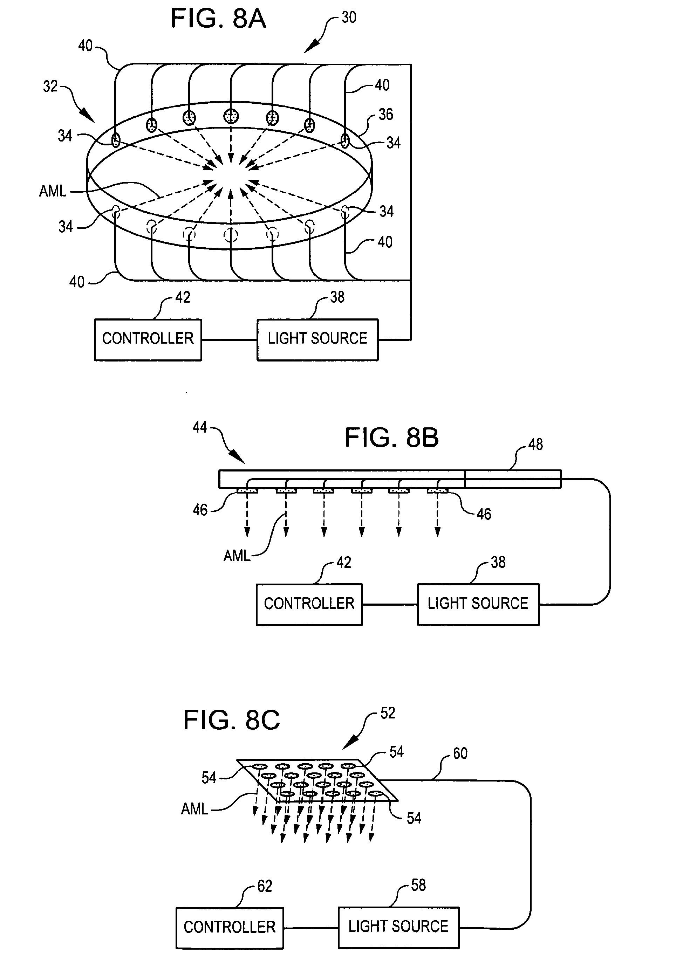

In some cases involving pathogens in tissue, such as those involving pathogens disposed deeper within a host tissue or where the specific location of the pathogens is not known, it may be advantageous to direct penetrating antimicrobial light at the pathogens from a variety of directions. **FIG. 8A** illustrates an antimicrobial light system **30** that includes a perimeter light applicator **32** that is designed to direct penetrating-antimicrobial light **(AML)** into a host tissue from a number of directions. The perimeter light applicator **32** can include a number of light emitters **34** disposed around a frame **36.** For example, the light emitters **34** can include sapphire. Sapphire may provide for good transmission at 1064 nm. While sapphire may be less flexible than quartz/silica fibers, sapphire may be stronger and/or more durable. The frame **36** can be configured to provide a desired central opening through which a host tissue can be positioned. For example, the frame can be appropriately configured (*e*.*g*., size, shape, etc.) for the treatment of a variety of host tissues, such as a patient's toe(s), foot, leg, torso, arm, hand, fingers, head, or any combination thereof. The light emitters **34** can be configured to emit light in desired directions towards a host tissue. For example, light emitters can be disposed around a circular frame and be confocally focused, such as towards the center of curvature of the circular frame. The perimeter light applicator can be configured with light emitters that can be redirected in different directions (*e*.*g*., through the use of an actuator(s)), which provides the ability to refocus the emitted light towards specific host tissue. The light emitters **34** can be coupled with a light source **38** by way of an optical coupling, such as optical fibers **40.** The light source **38** can be controlled, as discussed above, by a controller **41.** In some embodiments, the controller **41** can individually tailor the output of individual light emitters so as to apply a desired light treatment, such as energizing the light emitters in a desired sequence or with desired energy levels or pulse characteristics.

Various configurations of light applicators are possible. For example, as illustrated in **FIG. 8B****,** a light applicator **44** can include a one-dimensional array of light emitters **46** disposed on a wand **48** for applying antimicrobial light (**AML**). The wand **48** can have any desired shape so as to provide the desired spatial distribution and orientation of light emitters **46** for any desired treatment area on the host. For example, the wand **48** illustrated is straight, but can be curved so as to conform to the exterior shape of a host tissue. Such a wand applicator can be swept over a treatment area.

**FIG. 8C** illustrates a light applicator **52** that includes a two-dimensional array of light emitters **54.** A light applicator having a two-dimensional array of light emitters can be used to uniformly treat a corresponding two-dimensional treatment area without the need to move the light applicator. The light emitters **54** can be coupled with a light source **58** by way of an optical coupling, such as optical fibers **60.** A controller **62** can be coupled with the light source **58** and direct the light source **58** to energize the light emitters **54** in a desired pattern so as to maximize the destruction of the pathogen while minimizing any associated discomfort and/or damage induced in the host. For example, the controller **62** can be used to temporally separate energizing adjacent light emitters **54** so as to allow time for adjacent areas of the host tissue to cool between being subjected to light pulses.

To generate sufficient penetration necessary to destroy the targeted pathogens to the desired depth, it may be necessary to use an elevated power level. However, the use of an elevated power level may result in heating of host tissue to an uncomfortable level. In such a case, a cooling gel or liquid can be used between the light applicator and the host tissue. The penetrating-antimicrobial light can be transmitted through the cooling gel or liquid and the cooling gel or liquid can absorb heat from the surface of the host tissue.

Dentistry is another medical area where antimicrobial light can be used to combat surface pathogens and/or pathogens disposed within tissue. For example, antimicrobial light can be used to treat gingivitis, to treat herpes cold sores, for root canal sterilization, to treat a periapical abscess, and to treat jaw bone osteomyelitis. Light applicators can be configured (*e*.*g*., sized, shaped, etc.) that can be used to apply antimicrobial light for each of these applications.

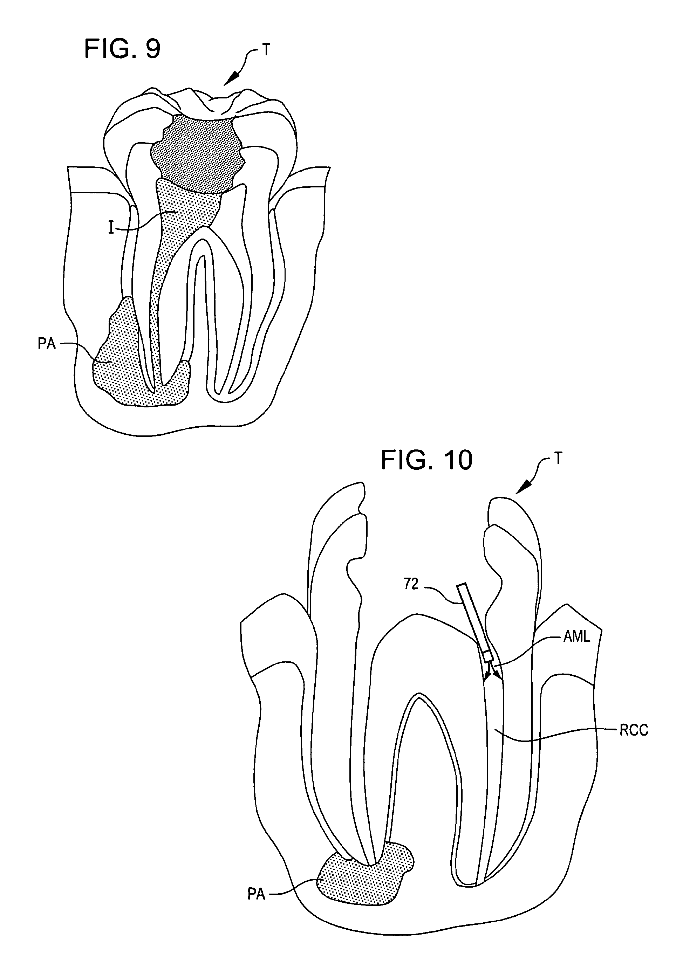

**FIG. 9** is a sectional representation of an infected tooth **(T),** showing infection **(I)** in the tooth and a periapical abscess **(PA).** **FIG. 10** illustrates the treatment of such a tooth **(T),** showing the tooth during a root canal procedure and the use of a light applicator **72** to administer antimicrobial light **(AML)** from within the root-canal channel **(RCC).** The light applicator **72** can be configured to be inserted into the root-canal channel **(RCC)** and can be moved along the channel so that antimicrobial light can be administered from within the periapical abscess **(PA)** and along the root-canal channel, thereby combating pathogens along the combined length of the abscess and the channel. The light applicator can include an optical fiber to transfer light from a light source to the point of application. To provide an area of coverage, the distal end of the optical fiber(s) can be scanned using know methods/actuators. Lenses can also be used to provide an area of coverage. An area of coverage can also be provided by using a length of exposed optical fiber (*e*.*g*., 1 to 2 mm or more), which can include a light scattering substance doped into the fiber tip.

Antimicrobial light can also be used to combat pathogens located within or adjacent to ear, nose, and throat cavities. Medical conditions that can be treated through the application of antimicrobial light within these cavities includes, but is not limited to, ear fungus, vocal cord warts, tonsillitis, laryngitis, sinus infections, and mucormycosis.

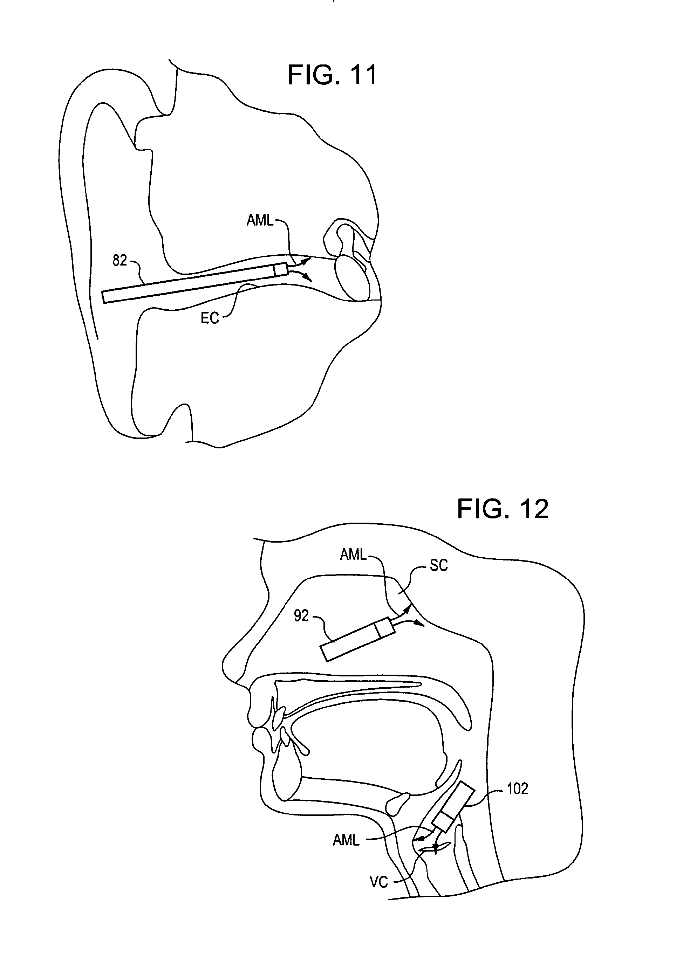

**FIG. 11** is a sectional representation of a human auditory system, illustrating the application of an antimicrobial light treatment from within an ear canal in accordance with an embodiment. A light applicator **82,** configured for insertion within an ear canal **(EC),** is used to apply antimicrobial light **(AML)** from within the ear canal **(EC).** The light applicator **82** can be moved along the ear canal to apply antimicrobial light along a length of the ear canal. A light applicator can be integrated with an imaging system, such as an endoscopic imaging system, which can be used by a treating professional during the application of the antimicrobial light treatment. A range of pathogens can be treated, such as ear fungus and bacterial infections.

**FIG. 12** is a sectional representation of internal cavities within a human head and neck, illustrating the application of antimicrobial light treatments from within the sinus cavity **(SC)** and to the vocal cords **(VC)** in accordance with an embodiment. A light applicator **92** is shown applying antimicrobial light **(AML)** from within the sinus cavity **(SC),** which can be used to combat a sinus infection. The light applicator **92** can be integrated with a rhinoscope, which can be a flexible fiber optic instrument. The light applicator **92** can be moved within the sinus cavity so that the antimicrobial light can be applied so as to cover a treatment area. A light applicator **102** is also shown applying antimicrobial light **(AML)** to the region of the larynx containing the vocal cords **(VC).** The light applicator **102** can be integrated with an endoscope, such as a bronchoscope. Antimicrobial light can be applied from within the illustrated cavities to treat a range of medical ailments, such as vocal cord warts, tonsillitis, laryngitis, and mucormycosis, etc.

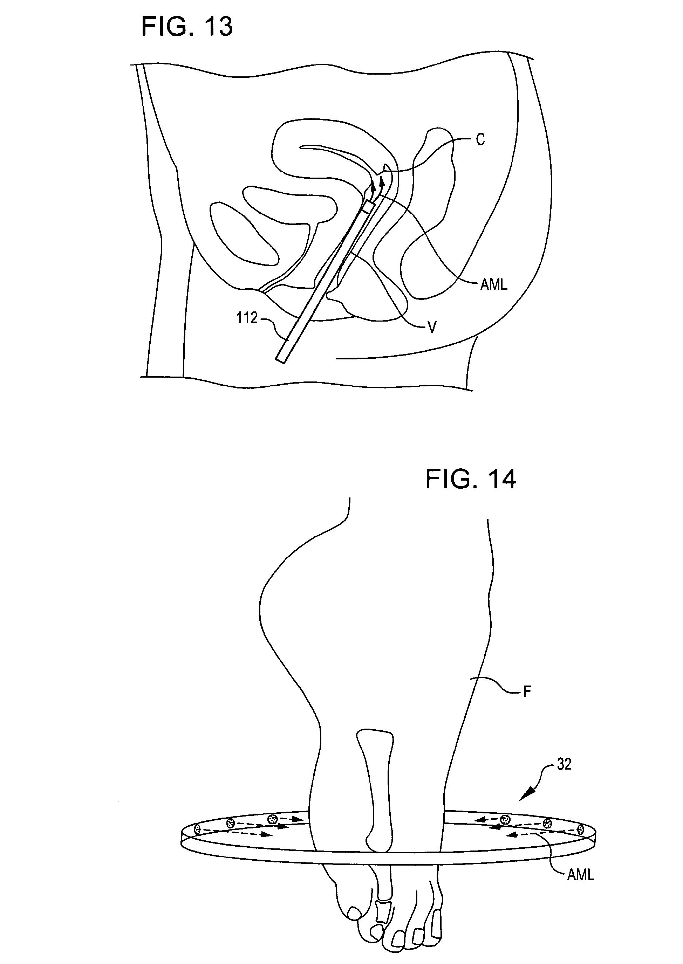

Antimicrobial light can also be used to combat pathogens located within or adjacent to the female reproductive system. Reproductive system conditions that can be treated through the application of antimicrobial light include, but are not limited to, genital herpes, genital warts, cervical warts, and yeast infections. **FIG. 13** is a sectional representation of a portion of a female patient, illustrating the application of an antimicrobial light treatment to the cervix **(C)** in accordance with an embodiment. A light applicator **112** can be used to apply antimicrobial light **(AML)** from within a cavity of the reproductive system, such as from within the vagina **(V).** The light applicator **112** can be integrated with endoscopes, such as a colposcope, a hysteroscope, a falloscope, an amnioscope, and/or a fetoscope.

Antimicrobial light can also be used to treat infection of the bone or bone marrow (osteomyelitis), which is usually caused by pyogenic bacteria or mycobacterial. **FIG. 14** illustrates the treatment of osteomyelitis within the foot **(F)** of a patient via the external application of antimicrobial light **(AML)** from multiple directions in accordance with an embodiment. A perimeter light applicator **32** (as discussed above with reference to **FIG. 8A****)** can be used to apply the treatment. By applying the antimicrobial light from multiple directions, the maximum light intensity seen by any portion of the host tissue (*e*.*g*., on the surface of the host tissue) can be limited while the pathogen(s) targeted are subjected to the combined energies of the antimicrobial light that reaches the pathogen(s) from the multiple directions. Also, the antimicrobial light can reach pathogen(s) even when the specific location of the pathogen(s) is unknown (*e*.*g*., the pathogen(s) responsible for plantar warts).

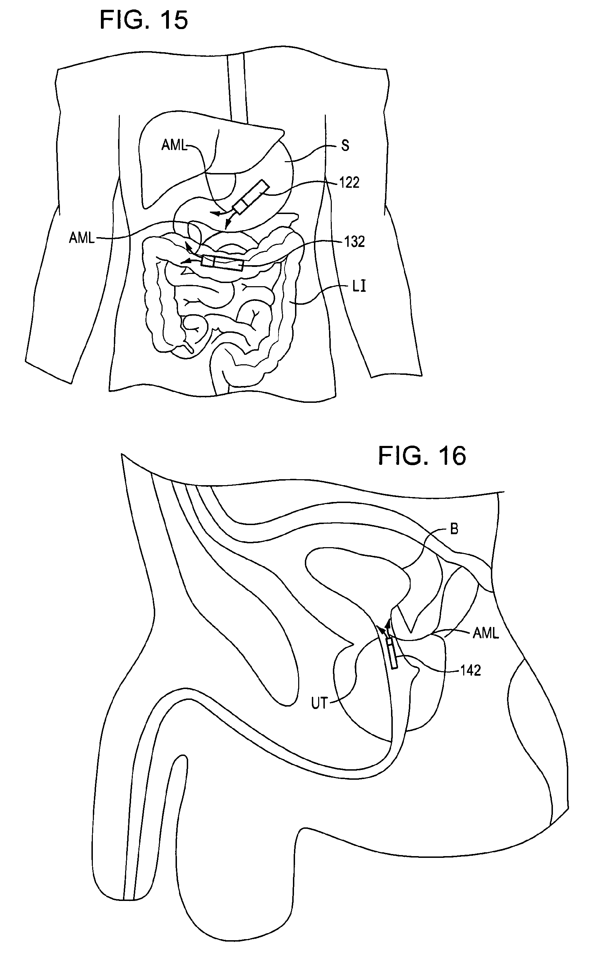

Antimicrobial light can also be used to combat pathogens within the digestive system of a patient. Digestive system conditions that can be treated through the application of antimicrobial light include, but are not limited to, bacterial ulcers and mucormycosis. **FIG. 15** illustrates the application of antimicrobial light treatments from within the stomach and from within the large intestine of a patient. A light applicator **122** is shown applying antimicrobial light **(AML)** to a pathogen(s) from within the stomach (S) (*e*.*g*., for the treatment of bacterial ulcers or mucormycosis). A light applicator **132** is also shown applying antimicrobial light **(AML)** to a pathogen(s) from within the large intestine **(LI).** The use of light applicators **122, 132** is not limited to the illustrated locations, but can be used throughout the digestive system. A light applicator **122, 132** can be integrated with an endoscope, such as one typically used during esophagogastroduodensocopy. A light applicator **122, 132** can be configured as a mobile autonomous camera and emitter.

Antimicrobial light can also be used to combat pathogens located within or adjacent to the urinary tract. Such pathogens include those responsible for various urinary tract and bladder infections (*e*.*g*., bacterial, viral, and fungal). **FIG. 16** illustrates the application of an antimicrobial light treatment from within the urinary tract in accordance with an embodiment. A light applicator **142** is shown applying antimicrobial light **(AML)** from where the urinary track **(UT)** meets the bladder **(B).** The light applicator **142** can be integrated with a cystoscope so that the treating professional can view the area to which the antimicrobial light is being applied. The light applicator **142** can be positioned along the urinary tract **(UT)** so as to cover a treatment area or treat different locations.

Antimicrobial light can also be used to treat a variety of infectious diseases. For example, antimicrobial light can be used to combat methincillin-resistant staphylococcus aureus (MRSA) and other hospital acquired infections using the above described devices and methods.

### Combating_Pathogens Within Bodily Fluids

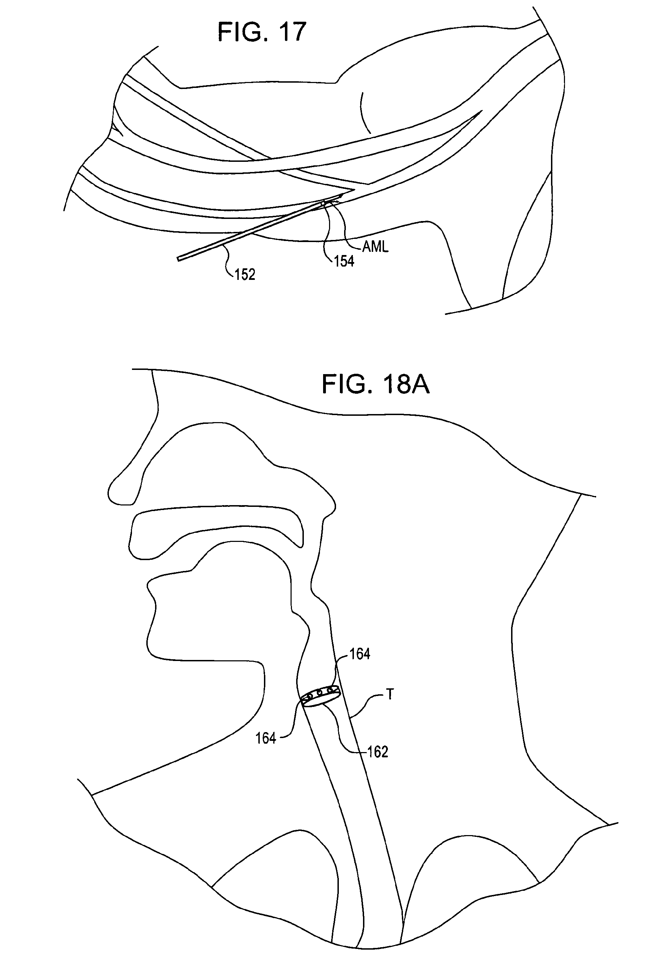

Application of antimicrobial light can be used to combat pathogens within bodily fluids. For example, antimicrobial light can be applied in-situ to treat blood-borne pathogens, such as hepatitis and other bacterial, viral, and fungal pathogens. **FIG. 17** illustrates the application of an in-situ antimicrobial light treatment from within a blood vessel in accordance with an embodiment. A light applicator **152** can be inserted into a blood vessel. The light applicator **152** can be configured, and the blood vessel selected, so that blood continues to flow in the blood vessel past a light-emitting end **154** of the light applicator **152.** While a range of blood vessels can be used, it may be advantageous to select a vein having a diameter compatible with the light applicator employed so as to avoid the increased chance of bleeding associated with arteries and to ensure an adequate flow of blood within the vein past the light-emitting end of the light applicator. With a continuous flow of blood past the light-emitting end, the antimicrobial light **(AML)** can be applied in a continuous, a varying, and/or a pulsed fashion. In either case, the intensity (and timing if pulsed) can be varied and/or selected so as to provide for sufficient intensity received by the pathogens, while avoiding damaging the host's blood and blood vessel. Due to the pulsing nature of the blood flow, a sensor can be included that monitors the flow rate of blood. Output from the sensor can be used to vary the intensity of the applied antimicrobial light and/or to time the delivery of applied light pulses. The treatment can be applied over a period of time so as to treat a desired quantity of the patient's blood. The treatment can also be applied from within multiple blood vessels so as to treat more blood in a reduced period of time. The treatment can be used to reduce (and possibly eliminate) the amount of the targeted pathogen(s) from the patient's blood.

### Combating Airborne Pathogens From Within a Patient

Antimicrobial light can be applied to treat airborne pathogens from within a patient. Various devices can be inserted within a respiratory duct so as to treat the air flowing within the duct with antimicrobial light. Various respiratory ailments can be treated with such an application of antimicrobial light, such as various pneumonias (*e*.*g*., bacterial, viral, and fungal), tuberculosis including multiple drug resistant tuberculosis, and mucormycosis.

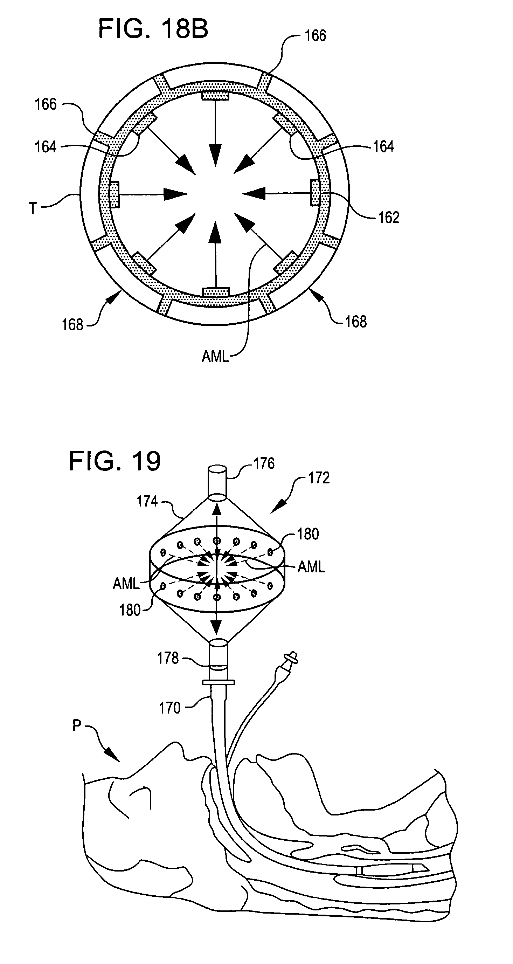

**FIGS. 18A** and **18B** illustrate one approach for the application of antimicrobial light from within a patient's respiratory duct for the treatment of airborne pathogens. A circular light applicator **162** can positioned within a patient's respiratory duct, such as a patient's trachea **(T).** The circular light applicator **162** can be configured such that, when installed, the air traversing the respiratory duct (*e*.*g*., trachea **(T)**) passes through a central opening of the circular light applicator **162.** Disposed around the perimeter of the light applicator **162** are a number of light emitters **164** that are configured and oriented to subject air passing through the central opening to a therapeutic dose of antimicrobial light **(AML).** The light emitters **164** can emit unidirectional light that is directed across the light applicator towards an opposing surface of the light applicator such that no light is directly targeted at trachea tissue. The surfaces of the light applicator can also be configured to reduce and/or eliminate reflection of antimicrobial light, thereby reducing and/or eliminating the amount of reflected light that is seen by trachea tissue. The light applicator can also be shaped to reduce the amount of light (either direct or reflected) seen by trachea tissue. For example, the light applicator can include edge flanges that block direct or reflected light from striking trachea tissue. When the light applicator **162** is configured to reduce and/or eliminate exposing host tissue to antimicrobial light, it may be possible to use ultraviolet antimicrobial light without the associated risk of cancer. Also, where bio-films on the inside of a respiratory track are targeted, the shallow penetration depth associated with UV light may allow for the application of UV antimicrobial light without damaging host tissue. Antimicrobial light that does not damage the host tissue can also be used.

The light applicator **162** can be configured to provide for the natural flow of mucous along the trachea **(T).** For example, as illustrated in **FIG. 18B****,** the light applicator **162** can include standoffs **166** so as to provide gaps **168** around the perimeter of the light applicator for the flow of the mucous. The light applicator can include a collection area(s) and a pump(s) so that mucous can be transferred to above the light applicator.

Various other approaches for the application of antimicrobial light within a patient for the treatment of airborne pathogens are possible. For example, a light applicator can be integrated with an endotracheal tube so that air traversing within the tube can be treated with antimicrobial light during use.

### Combating Airborne Pathogens Entering/Leaving the Respiratory System

**FIG. 19** illustrates the application of antimicrobial light **(AML)** to airborne pathogens prior to entering and/or after leaving the respiratory system of a patient **(P).** The antimicrobial light can be applied by a light applicator that is coupled with an interface component (*e*.*g*., a breathing mask, an endotracheal tube **170** as shown, etc.), through which a patient **(P)** at least partially breathes. Similar to the light applicators **12, 32, 44, 52** (shown in **FIGS. 2****,** **8A, 8B** and **8C****,** respectively), the light applicator can be coupled with a light source that supplies antimicrobial light to the light applicator and the light source can be coupled with a controller for controlling the operation of the light source. The light applicator can be configured to apply antimicrobial light to airborne pathogens transferred to and/or received from the interface component. The light applicator can be used to apply antimicrobial light having a wavelength(s) selected from a range of wavelengths, such as from ultraviolet and/or non-ultraviolet wavelengths. The light applicator can be configured to shield the patient and/or other persons from being exposed to the antimicrobial light, such as by applying the antimicrobial light to the pathogens from within an enclosed housing having an inlet and an outlet. The antimicrobial light application can also be accomplished using coherent light, which can be directed so as to reduce or eliminate exposure of the patient and/or other persons. Such shielding can be used with any antimicrobial light, but may be particularly beneficial when used in conjunction with antimicrobial light that includes an ultraviolet wavelength(s).

Various approaches for applying antimicrobial light to airborne pathogens prior to entering and/or after leaving the respiratory system of a patient are possible. For example, the light applicator **172** shown in **FIG. 19** includes a housing **174** having a first port **176** and a second port **178.** Similar to the light applicator **30** shown in **FIG. 8A****,** the light applicator **172** includes a plurality of light emitters **180** that are oriented to direct antimicrobial light **(AML)** towards the center of the light applicator. Another approach involves directing antimicrobial light across a region where the air and/or oxygen entering and/or leaving the patient **(P)** is traveling (*e*.*g*., by using an arrangement of light emitters as illustrated in the light applicators **44**, **52** shown in **FIGS. 8B** and **8C**, respectively). Additionally, existing systems (*e*.*g*., ventilators, sleep apnea machines, etc.) can be configured so that antimicrobial light can be applied to airborne pathogens prior to entering and/or after leaving the respiratory system of a patient.

### Methods and Systems for Automated Treatment

Methods and systems for automated treatment of pathogens using antimicrobial light are provided. Such automated methods and systems can be beneficial in the treatment of a wide range of pathogens, and can be especially beneficial for the treatment of toenail fungus. While disclosed embodiments are discussed primarily in the context of the treatment of toenail fungus, a person of skill in the art will appreciate that such methods and systems can be adapted for use with other pathogens, for example, the above discussed pathogens.

The disclosed methods and systems for automated antimicrobial light treatment provide numerous benefits. In many embodiments, an automated scan pattern is used, which may provide for a more uniform application of antimicrobial light. The use of an automated scan pattern may significantly reduce the amount of time and/or effort required on the part of the treating professional, and may thereby help to reduce the cost of the treatment. In many embodiments, standardized imaging conditions are used to capture images during different treatment sessions, which can be compared to evaluate treatment progress. In many embodiments, standardized imaging conditions are used during the capture of images that are processed to identify areas to be treated, for example, by using an automated processing approach such as a pattern-recognition algorithm, an edge-detection algorithm, and/or a manual approach such as user identification of areas to be treated. In many embodiments, a composite image of the area to be treated is displayed that includes a representation of a candidate treatment scan pattern. The user can then accept or modify the candidate treatment scan pattern prior to the application of the treatment. In many embodiments, a manual light applicator is provided. A manual light applicator may provide increase flexibility for certain treatment situations. In many embodiments, a holding fixture is used to position and/or stabilize the area to be treated relative to an automated light scanner, which may result in a more consistent treatment application. A holding fixture can include a background surface selected to enhance image processing using, for example, a known pattern-recognition approach, a known edge-detection algorithm, etc., by contributing to the standardization of the imaging conditions.

Although not known for certain, the treatment of a toenail fungus described throughout this document is believed to be fungi-static as opposed to fungicidal. That is, the treatment is believed to stop the growth of the toenail fungus as opposed to killing the toenail fungus. By stopping the growth of the toenail fungus, the growth rate of the toenail results in a gradual clearing of the toenail fungus from the toenail bed.

In many embodiments, such methods and systems are embodied in treatment systems subject to treatment based license fees. Accordingly, in many embodiments, a method for treating a patient with a licensed treatment system is provided, which may help to reduce the amount of overhead expenses associated with such a licensed systems, for example, overhead expenses associated with the processing of license fee payments, as well as overhead expenses associated with verification of license fee agreement compliance.

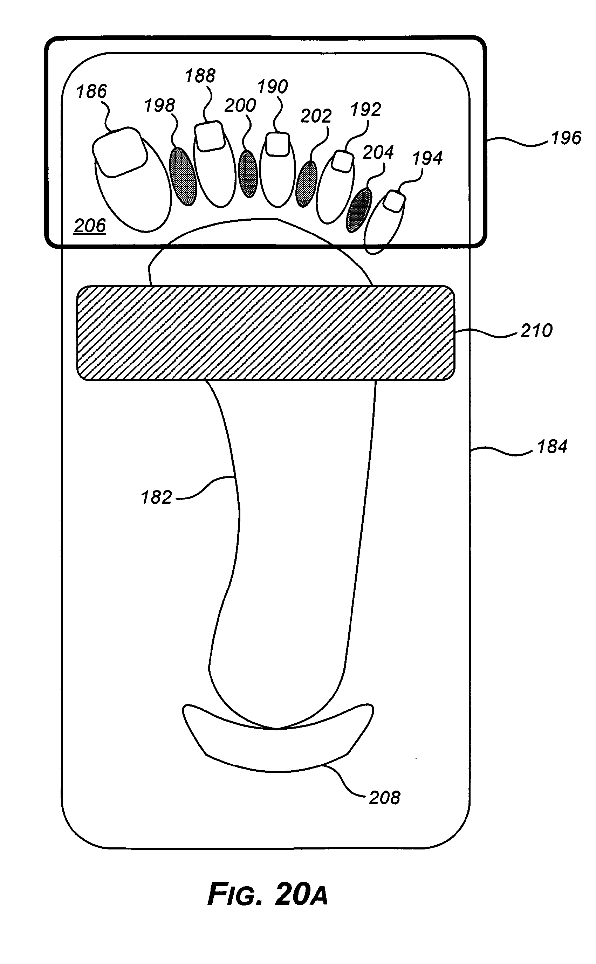

Turning now to the drawing figures, **FIG. 20A** is a schematic diagram of a patient foot **182** engaged with a holding fixture **184** configured to receive and position the patient's foot **182** so as to position the patient's toenails **186, 188, 190, 192, 194** relative to a light scanner **196,** in accordance with many embodiments. The holding fixture **184** includes toe separation features **198, 200, 202, 204,** a background surface **206**, a heel support **208,** and a strap **210.** The toe separation features **198, 200, 202, 204** are configured to position the toes and separate the patient's toes. By separating the toes, a plan-view image of the toes will have the toes at least partially outlined by the background surface **206,** which may provide for an image that can be more reliably processed using a known automated pattern-recognition approach, for example, a known edge-detection algorithm. Each toenail can be associated with an identifier, for example, the right big toenail **186** illustrated can be identified as R1, toenail **188** as R2, toenail **190** as R3, toenail **192** as R4, toenail **194** as R5, and similar identifiers L1, L2, L3, L4, and L5 can be used for the patient's left-foot toenails (not shown). Different and/or adjustable toe separation features can be used to accommodate different feet sizes. The background surface **206** can be selected to enhance image processing, for example, by comprising a color and/or pattern that contrasts with the patient's toes and/or toenails and/or is more easily detected by the image-processing approach used. In use, a patient's foot can be placed into engagement with the holding fixture so as to position the patient's toes as illustrated, and the heel support **208** and the strap **210** can be used to secure the patient's foot in the holding fixture **184.**

To facilitate the engagement of the patient's foot with the holding fixture **184,** the holding fixture and the light scanner **196** can be positioned relative to each other after the patient's foot has been engaged with the holding fixture. For example, the light scanner can be coupled with the holding fixture via a mechanism that provides the ability to move the light scanner out of the way while the patient's foot is being engaged with the holding fixture. A person of skill in the art will appreciate that a wide range of approaches for positioning the light scanner relative to the holding fixture can be used.

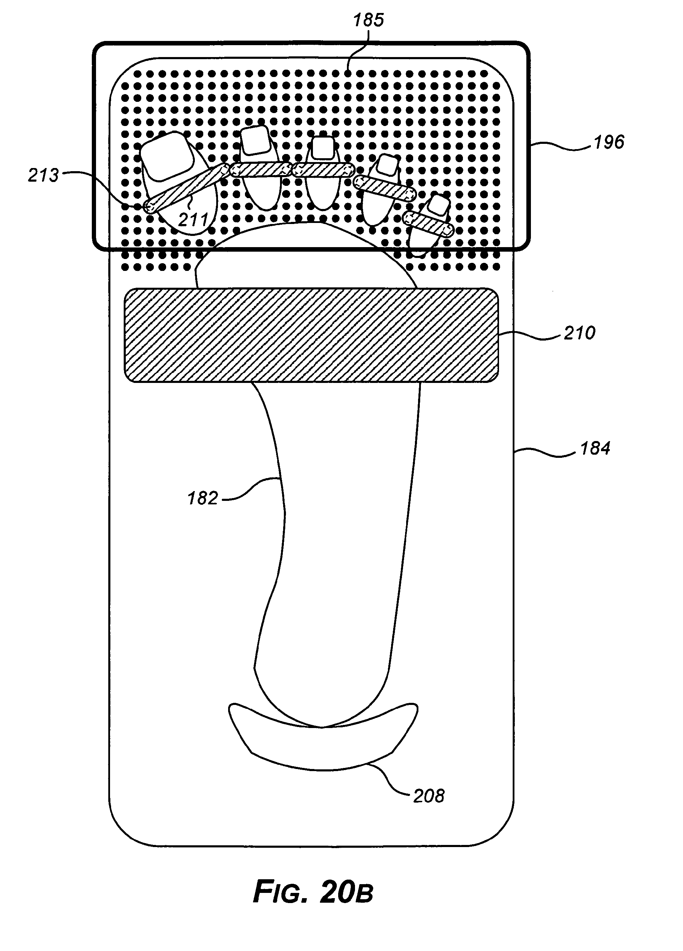

**FIG. 20B** is a schematic diagram of a patient's foot engaged with a holding fixture having toe straps **211** used to position the patient's toenails relative to the light scanner **196,** in accordance with many embodiments. The holding fixture **184** can include a pattern of receptacles **185** configured to accept and secure mating pegs **213** of the toe straps **211** so as to hold the patient's toenails fixed relative to the holding fixture **184.** Each of the toe straps **211** can be placed, for example, spaced apart from the cuticle so as to not prevent application of light to the non-visible portion of the toenail that has not yet emerged from the cuticle while still being placed between the cuticle and the first joint of the toe. The toe straps **211** can be configured to enhance image processing, for example, by comprising a color and/or pattern that contrasts with the patient's toes and/or toenails and/or is more easily detected by the image-processing approach used. For example, the location of the toe straps **211** can be detected and used to locate the toenail areas to be treated. The pattern of receptacles **185** can be used to accommodate a range of sizes of feet and/or toes.

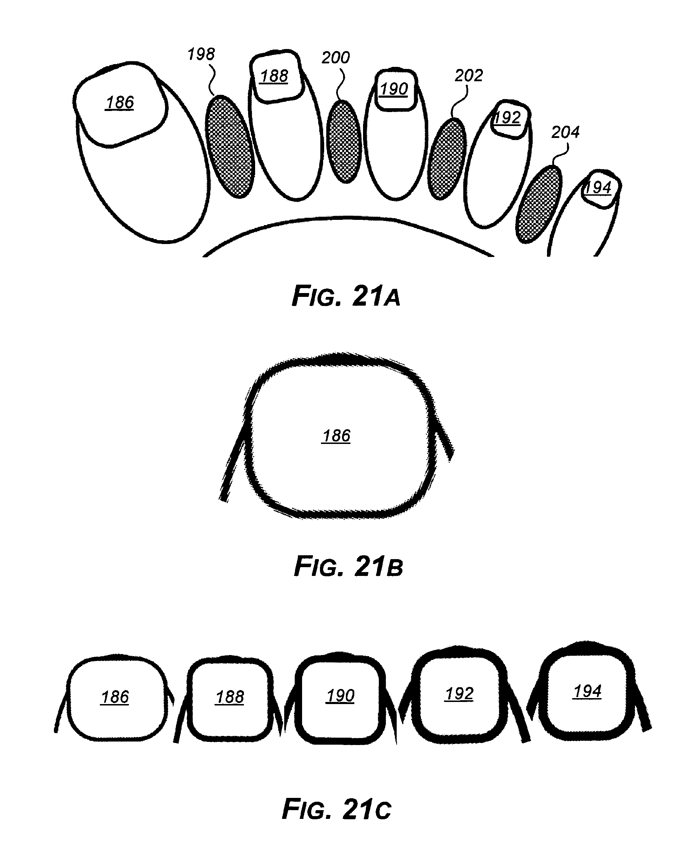

**FIG. 21A** is a schematic diagram illustrating a patient's toes separated by the toe separation features of the holding fixture of **FIG. 20** and corresponding to a field-of-view of the light scanner of **FIG. 20**, in accordance with many embodiments. An imaging device can be used to capture an image that includes the patient's toenails, for example, an image similar to the illustration of **FIG. 21A****.** As discussed above, the image can be processed using a known approach (*e*.*g*., a known pattern-recognition approach, a known edge-detection approach, etc.) to identify individual toes and/or toenails. In addition to the holding fixture background **206,** the toe separation features **198, 200, 202, 204** can be configured to enhance image processing, for example, by having a color, pattern, and/or shape selected to enhance recognition of the toe separation features during image processing. The position of the holding fixture relative to the imaging device and/or the light scanner can be used to at least partially identify the location of the toes and/or toenails relative to the imaging device and/or the light scanner. The imaging device can be coupled with the light scanner so as to provide a fixed positional relationship between the imaging device and the light scanner. The imaging device and the light scanner can be calibrated so as to provide a correspondence between image positions and scanned light positions.

A treatment system can include a display that can be used to display various images to a treatment system user, for example, areas to be treated or that have been treated. Such images can be enlarged, reoriented, and/or combined with images of other treatment areas. For example, **FIG. 21B** illustrates the display of an image of a patient's toenail (in this instance an image of the large toenail of **FIGS. 20** and **21A****,** in accordance with many embodiments. An image of an individual toenail, such as illustrated in **FIG. 21B****,** can be extracted from a more extensive image, such as illustrated in **FIG. 21A****,** and then be scaled and/or reoriented. **FIG. 21C** illustrates the display of multiple toenail images showing five of a patient's toenails, the toenail images being scaled to approximately the same size and reoriented, in accordance with many embodiments. Such a rescaled, composite image display may be useful in planning and/or evaluating treatments.

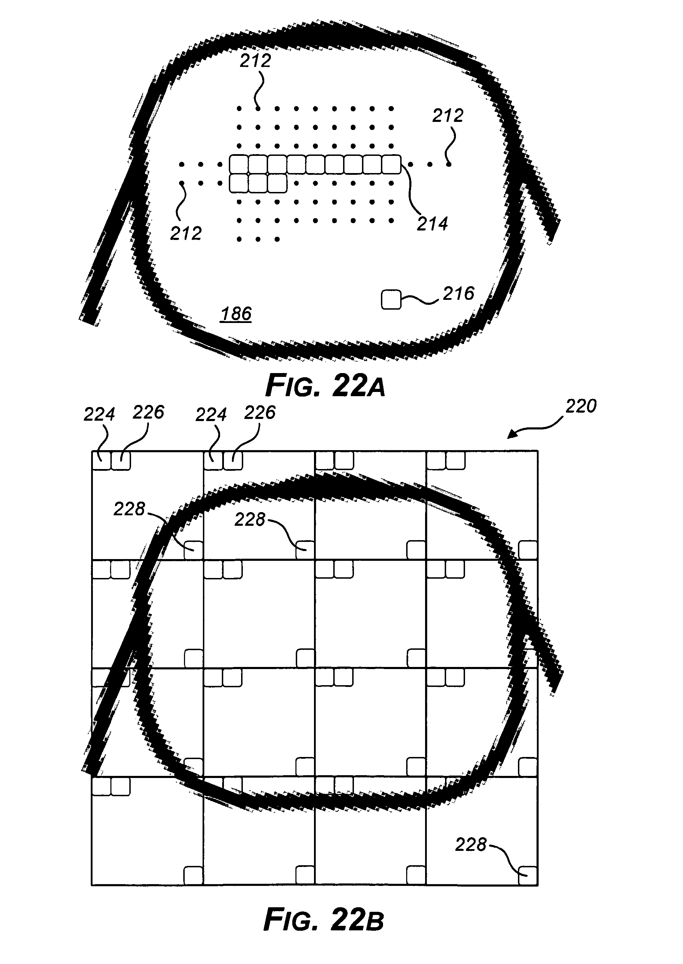

In many embodiments, a composite image is displayed that includes an image of a toenail and an overlaid representation of a candidate scan pattern. **FIG. 22A** illustrates such a composite image. The scan pattern representation used can reflect the characteristics of the antimicrobial light used in the scan pattern, for example, whether the light is pulsed or continuously emitted, intensity, duration, scan rate, size, etc. The scan pattern representation can be shown in a variety of ways, for example, as a collection of scan positions **212** designating the center of individual light pulses, or as a series of subareas **214** designating subareas targeted by individual light pulses. A treating professional can review the candidate scan pattern to determine whether the candidate pattern is acceptable as is, or should be modified. In many embodiments, a treatment system includes a user interface that provides for the modification of the candidate scan pattern by a treating professional, for example, via an interactive modification and/or a non-interactive approach. The modified scan pattern can be displayed with a scan pattern representation as discussed above, which can include, for example, one or more user designated targeted subareas **216.** The modified scan pattern can be displayed for acceptance or further modification.

In many embodiments, the system displays a scan pattern representation that is updated during the application of the treatment so as to show the progress of the treatment. For example, in a scan pattern representation that includes a series of targeted subareas **214** as shown in **FIG. 22A****,** a characteristic of individual subareas **214** can be changed in response to that subarea being treated, for example, by changing a color of the subarea **214** symbol, or by coloring in the subarea.

In many embodiments, the system includes a tracking mechanism that tracks the position of the patient's toenail during the treatment session. An image based tracking mechanism can be implemented using a known method, such as by tracking the location of one or more discernible features during a treatment session (*e*.*g*., natural features of the toenail and/or toe, reference features added to the toenail and/or toe for tracking purposes, etc.). The resulting positional data can be used to correct the locations targeted by the light scanner to account for any motion of the toenail.

**FIG. 22B** illustrates a scan pattern **220** in which a sequence of pulses are spaced apart during the scan pattern **220** so as to limit thermal accumulation, in accordance with many embodiments. In the scan pattern **220,** a scan area **222** is divided into sixteen subareas via four rows and four columns. More or less subareas may be used, and such subareas may be squares, rectangles, or any desired shape. Light is scanned over the scan area **222** by sequentially applying an individual bursts to each subarea. For example, a first pattern of bursts **224** are sequentially applied to each subarea followed by a second pattern of bursts **226** sequentially applied to each subarea. The scan pattern progresses in a like fashion until a final pattern of bursts **228** are sequentially applied to each subarea to complete the scan pattern. The scan pattern 220 provides increased temporal separation between bursts applied to adjoining locations, thereby providing increased time for thermal dissipation prior to the application of a subsequent burst to an adjoining location. In many embodiments, the burst is suppressed when the location targeted is located outside a desired treatment area (*e*.*g*., outside the visible portion of the nail and outside the non-visible portion of the nail that has not yet emerged from the cuticle).

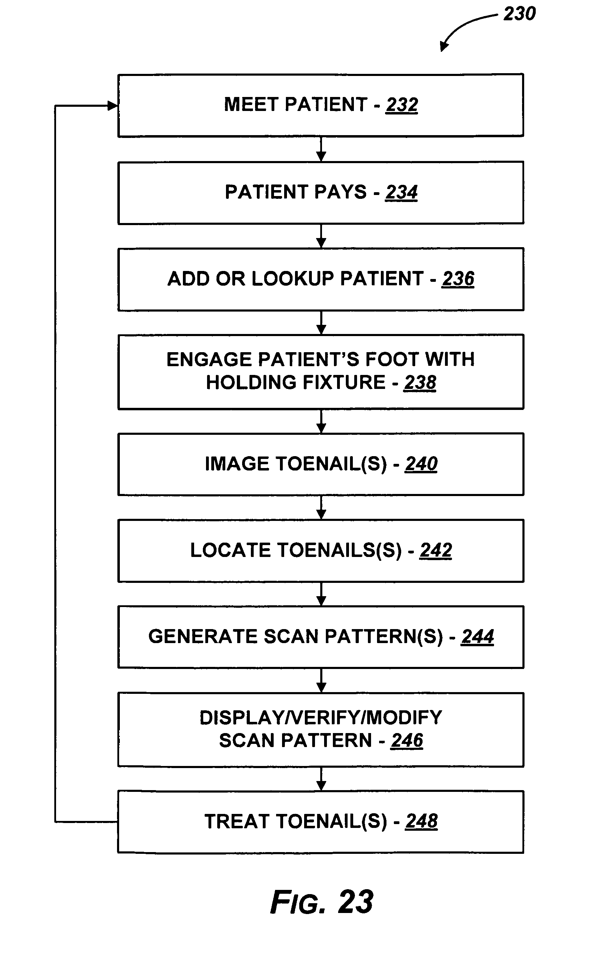

**FIG. 23** is a flow chart of a method **230** for treating toenail fungus, in accordance with many embodiments. The illustrated steps include optional steps. In step **232,** a treating professional or associate meets the patient. In step **234,** the patient pays for the treatment. In step **236,** the treating professional or associate can access patient records for existing patients or add records a new patient. In step **238,** the patient's foot (or feet) are engaged with a holding fixture(s), for example, a holding fixture in accordance with the holding fixture **184** illustrated and discussed above (see, *e*.*g*., **FIGS. 20A** and **20B****).** In step **240,** an image of the patient's toenail(s) is obtained. In step **242,** the patient's toenail(s) to be treated are located. The location of a toenail(s) can be determined in various ways. For example, the image obtained in step **240** can be processed to identify a toenail area(s) using a known image-processing approach such as a known pattern-recognition approaches and/or a known edge-detection approach. The image obtained in step **240** can also be displayed and a user can designate an area(s) of the image corresponding to a toenail(s) and/or a treatment area of a toenail(s). In step **244,** a candidate scan pattern is automatically and/or manually generated for a toenail area(s). In step **246,** the candidate scan pattern is displayed for user acceptance or modification. In step **248,** the toenail is treated using the resulting scan pattern.

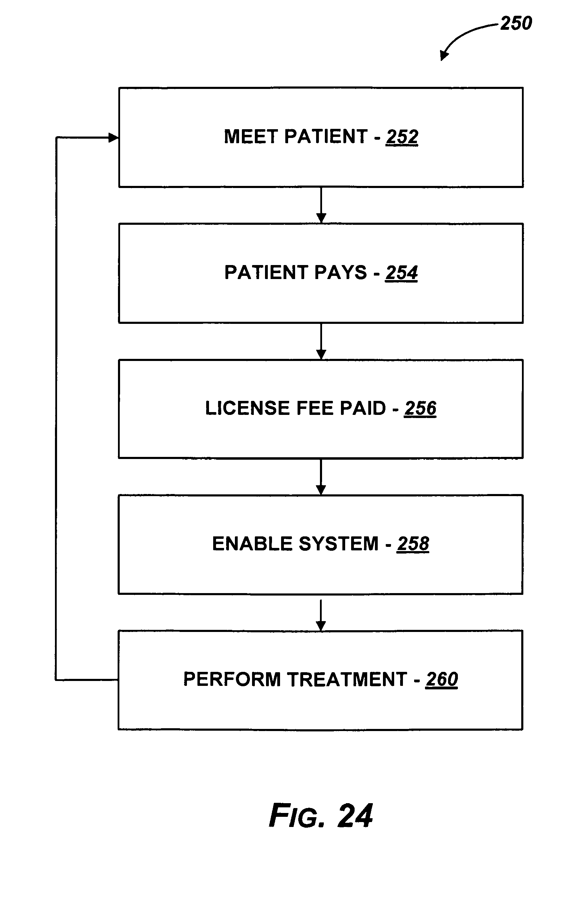

**FIG. 24** is a flow chart of a method **250** for treating a patient with a licensed treatment system, in accordance with many embodiments. In step **252,** a treating professional or associate meets the patient. In step **254,** the patient pays for the treatment. In step **256,** in response to the patient's payment, the license fee is paid. In step **258,** in response to the license fee payment, the licensed treatment system is enabled, for example, via receiving a communication that is used to enable the licensed treatment system. In step **260,** the licensed treatment system is used to treat the patient.

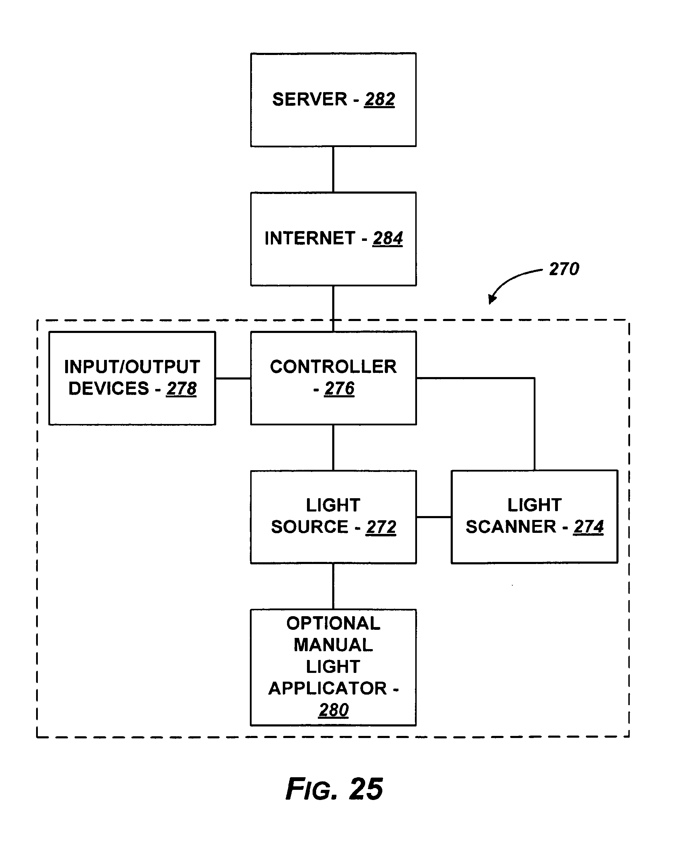

**FIG. 25** is a simplified block diagram of an automated antimicrobial light treatment system **270,** in accordance with many embodiments. The system **270** includes an antimicrobial light source **272,** a light scanner **274,** a controller **276**, input/output devices **278,** and an optional manual light applicator **280.** In many embodiments, the system **270** is configured to communicate with a server **282** via a communication link **284,** for example, the internet. In many embodiments, an authorizing communication is received from the server **282** in response to a license fee payment. In many embodiments, the authorizing communication is used to enable the treatment system for a treatment session.

In many embodiments, the antimicrobial light source **272** is operable to generate light with a wavelength corresponding to an absorption spectrum for the targeted pathogen(s). In many embodiments, the light source **272** is a laser, for example, lasers as describe above with regard to the light source **14** discussed above (ref. **FIG. 2****).** In many embodiments, the light source **272** generates a single continuously-emitting beam or multiple continuously-emitting beams. In many embodiments, the light source **272** generates a single pulsed beam or multiple pulsed beams.

The light source **272** is operatively coupled with the light scanner **274** so as to transfer the generated light beam(s) to the light scanner via one or more optical path. In many embodiments, the one or more optical path comprises at least one of a mirror, a lens, or an optical fiber. Multiple optical paths can be used where the light source **272** generates multiple beams, for example, multiple optical fibers (*e*.*g*., a multi-fiber trunk cable) can be used to transfer the multiple beams from the light source **272** to the light scanner **274.**

The light scanner **274** is operable to scan the received light beam(s) in a scan pattern so as to irradiate a target area of the targeted tissue and/or targeted pathogen. In many embodiments, the light scanner **274** comprises at least one scan head to scan the light beam in one or more dimensions and/or optical components to direct and/or focus the light beam(s). In many embodiments, a scan head comprises at least one galvanometric mirror operable to scan the light beam in at least one dimension. In many embodiments, the light scanner **274** comprises at least one of an x-direction motion stage, a y-direction motion stage, or a z-direction motion stage so to reposition at least one beam output (*e*.*g*., a scan head, an optical fiber, etc.) in one, two, or three dimensions. In many embodiments, the light scanner **274** comprises a power meter for measuring beam energy, for example, by measuring a portion of a segment of the beam or a reflection of the segment of the beam prior a target area being irradiated by the beam segment, and/or by measuring the segment of the beam by its reflection from the target area.

In many embodiments, the light scanner comprises at least one imaging device for capturing an image(s) (*e*.*g*., of the patient's toes, toenails, toenail fungus, etc.). In many embodiments, an imaging device has a known positional relationship relative to the light scanner. In many embodiments, an imaging device is integrated with the scan head so that the imaging device can capture an image that includes an area targetable by the scan head.

The controller **276** is operatively coupled with the light source **272** and the light scanner **274** so as to control the operation of the light source and the light scanner. The controller can be coupled with the light source and/or the light scanner for unidirectional communication (*e*.*g*., from the controller to the light source and/or to the light scanner) or for bidirectional communication (*e*.*g*., using a USB cable between the controller and the light source and/or the light scanner). The use of a bidirectional communication link can be used, for example, to transfer images captured by an imaging device to the controller for processing, storage, and/or display.

In many embodiments, a captured image can be processed by the controller **276** using a known approach (*e*.*g*., a pattern-recognition algorithm, an edge-detection algorithm, manual user area designation, etc.) to identify toenail areas for treatment. For example, an image such as the image illustrated in **FIG. 21A** can be processed using known approaches to identify toenail areas **186, 188, 190, 192, 194.** The position of the identified toenail areas relative to the light scanner can be determined based on the location of the areas within the image. The light scanner can be calibrated so that an image of a toenail can be used to determine corresponding light scanner control inputs necessary to accurately target the toenail.

In many embodiments, at least one captured image is transferred to the controller **276** for storage and/or display. In many embodiments, at least one captured image is displayed for evaluation of the toenail fungus. In many embodiments, at least one captured image captured during a treatment session is stored for later evaluation relative to an image captured during a subsequent treatment session. Such evaluation can be accomplished using automated image evaluation techniques, for example, by evaluating an image of a toenail fungus infected toenail relative to a non-infected toenail standard or relative to standards representative of different levels of toenail fungus infection. Such evaluation can also be accomplished subjectively, for example, by displaying an image next to a previous image or a standard image for evaluation by a treating professional.

In many embodiments, the controller **276** comprises components typically found in personal computer systems. For example, the controller can comprise at least one processor that communicates with a number of peripheral devices via a bus subsystem. These peripheral devices typically include a storage subsystem. The storage subsystem maintains the basic programming and data constructs that provide the functionality of the controller. Software modules for implementing the processing discussed above are typically stored in the storage subsystem. The storage subsystem typically includes a memory subsystem and a file storage subsystem. The memory subsystem typically includes a number of memories including a main random access memory (RAM) for storage of instructions and data during program execution and a read only memory (ROM), in which fixed instructions are stored. The file storage subsystem provides persistent (non-volatile) storage for program and data files, and can include a hard drive, a disk drive, or other non-volatile memory such as a flash memory. An input device, for example a disk drive, can be used to input the software modules discussed above. Alternatively, any other means well know in the art may be used to input the software modules, for example, a USB port. In this context, the term "bus subsystem" is used generically so as to include any mechanism for letting the various components and subsystems communicate with each other as intended. The bus subsystem typical system has a number of buses such as a local bus and one or more expansion buses (*e*.*g*., ADB, SCSI, ISA, EISA, MCA, NuBus, or PCI), as well as serial and parallel ports.

In many embodiments, the controller **276** is operatively coupled with input/output devices **278,** for example, a keyboard, pointing device, a display, a printer, and/or other known input output devices. These input/output devices provide an interface by which a treating professional can control the operation of the system to treat a patient.

In many embodiments, the system **270** additionally provides a non-automated treatment mode. For example, the system **270** can include an optional manual light applicator **280** that can be used by a treating professional to manually apply treatment, for example, such as the light applicator **12** discussed above with reference to **FIG. 2****.** As another example, the controller **276** can display an image of a patient's toenail and a treating professional can manually target individual locations for the application of antimicrobial light pulses by the light scanner **274.**

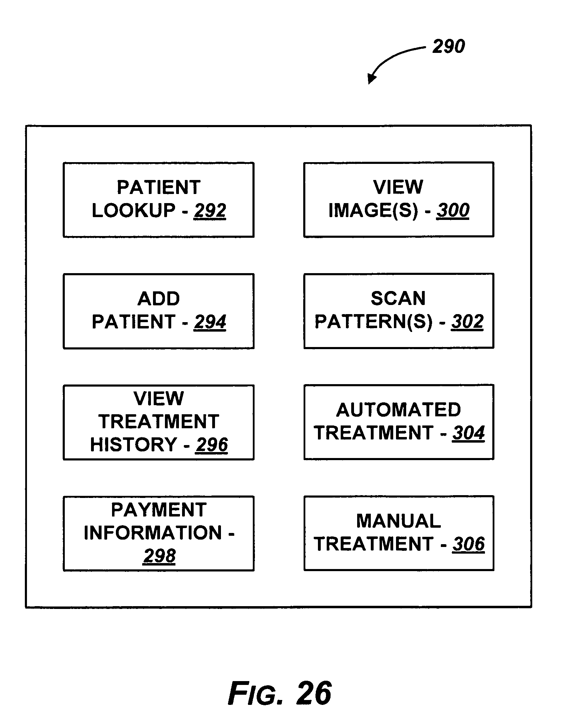

In many embodiments, a treating professional interacts with the system **270** via a user interface that provides the treating professional with the ability to accomplish desired functions. For example, **FIG. 26** is a simplified block diagram of functionality that can be accessed through a user interface **290** of an automated antimicrobial light treatment system, in accordance with many embodiments. The user interface **290** provides for the access to functionality for patient lookup **292,** for adding a patient **294,** for viewing a patient's treatment history **296,** for viewing patient payment information **298,** for viewing images **300** (*e*.*g*., current or previous images of the patient's toenails), for scan patterns **302** (*e*.*g*., selecting a scan pattern, viewing a scan pattern overlaid on an image of a patient's toenail, modifying a scan pattern, accepting a scan pattern, etc.), for automated treatment **304** (*e*.*g*., setting treatment parameters such as pulse energy, pulse shape, pulse duration, and/or pulse spacing; commencing an automated scan pattern treatment; monitoring the progress of an automated scan pattern treatment; stopping an automated scan pattern treatment; etc.), and for manual treatment **306** (*e*.*g*., enabling an attached manual light applicator such as the optional manual light applicator **280** discussed above with reference to **FIG. 25****,** for accomplishing a manual mode treatment via the light scanner as discussed above with reference to **FIG. 25****,** etc.). It should be appreciated that known approaches can be used to configure a user interface that can be used by a treating professional to interact with the system **270,** for example, the use of a graphical user interface, a touch screen, pull down menus, etc.

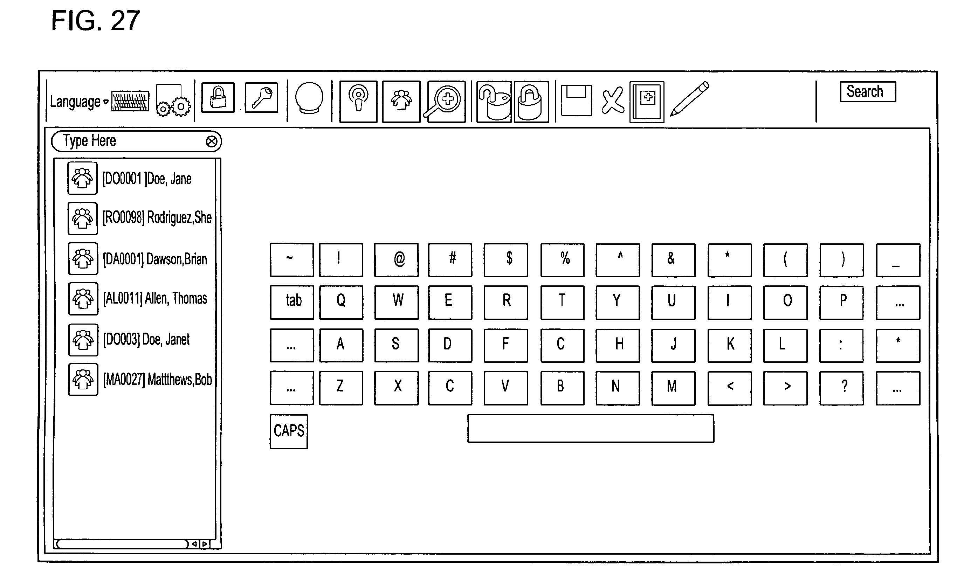

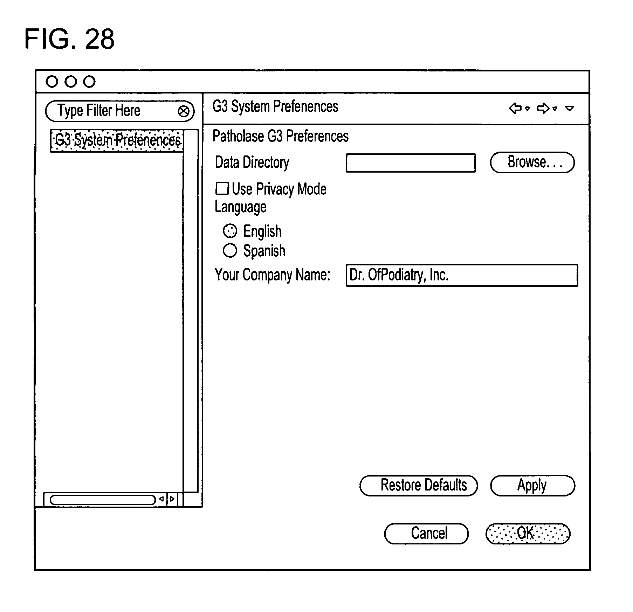

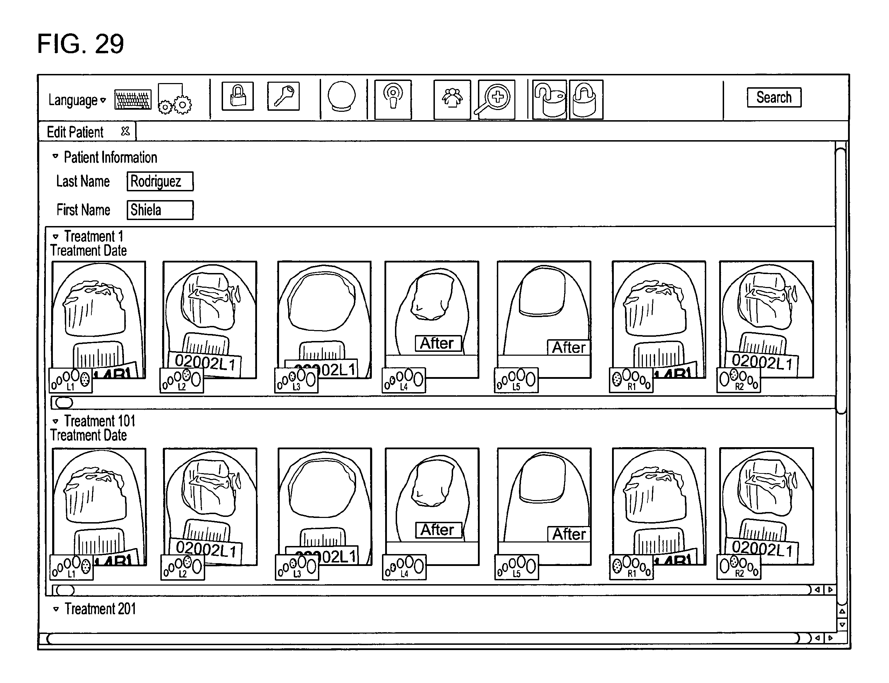

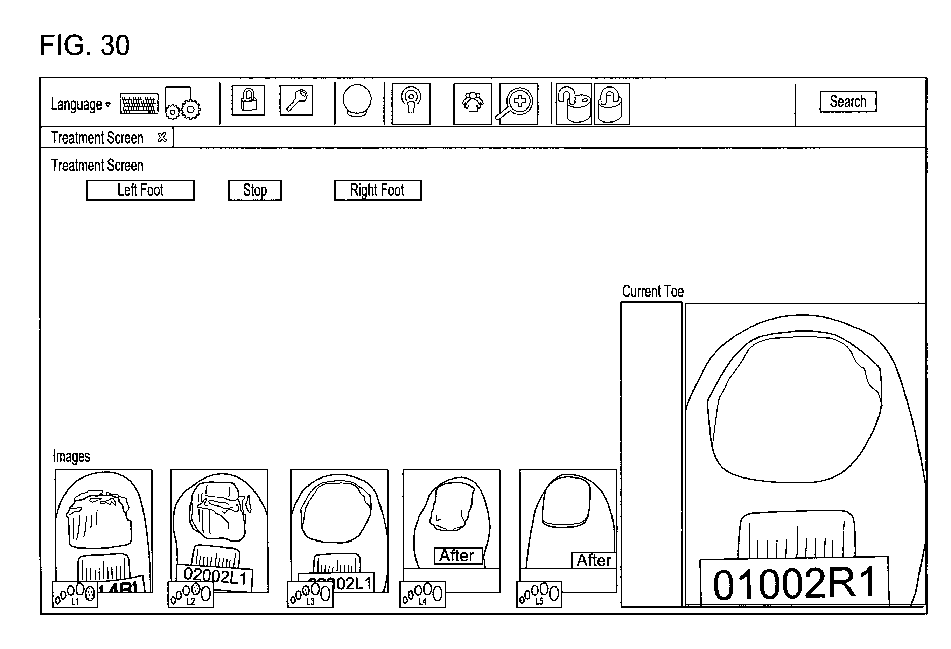

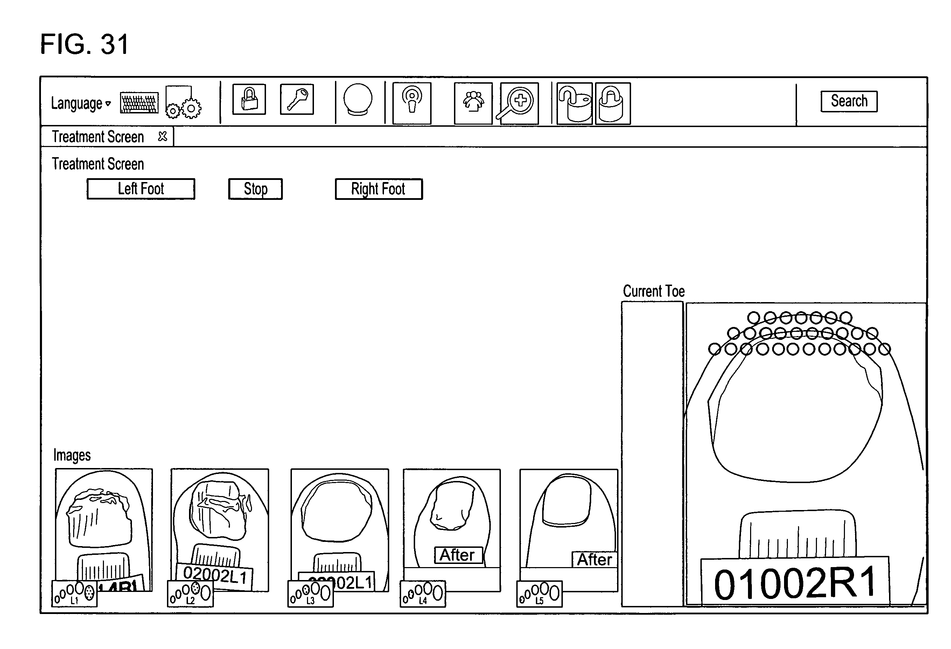

**FIGS. 27****,** **28****,** **29****,** **30****,** and **31** show example graphical user interface screens for a toenail fungus treatment system having a touch-screen display, in accordance with many embodiments. **FIG. 27** shows an existing patient selection screen (see related discussion above for patient lookup **292** discussed above with reference to **FIG. 26****),** which can be used to access records for the patient, including images of the patient's toenails captured during prior treatment sessions. **FIG. 28** shows a screen for displaying and/or setting system preferences. **FIG. 29** shows a screen for displaying images of a patient's toenails from one or more treatment sessions (see related discussion above for **FIGS. 21B, 21C****,** and **26****).** **FIG. 30** shows a screen for selecting a patient's toenail for treatment (see related discussion above for **FIGS. 21B, 21C****,** **23****,** **25****,** and **26**). **FIG. 31** shows a screen displaying a scan pattern overlaid on a patient's toenail (see related discussion above for **FIG. 22A****,** **23****,** **25****,** and **26****).**

Other variations are within the scope of the present invention. Thus, while the invention is susceptible to various modifications and alternative constructions, a certain illustrated embodiment thereof is shown in the drawings and has been described above in detail. It should be understood, however, that there is no intention to limit the invention to the specific form or forms disclosed, but on the contrary, the intention is to cover all modifications, alternative constructions, and equivalents falling within the scope of the invention, as defined in the appended claims.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (*i*.*e*., meaning "including, but not limited to,") unless otherwise noted. The term "connected" is to be construed as partly or wholly contained within, attached to, or joined together, even if there is something intervening. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e*.*g*., "such as") provided herein, is intended merely to better illuminate embodiments of the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A system (10) for treating nail disease in a patient, the system comprising:
a light applicator (12) operable to irradiate a nail having a nail disease with light comprising a duty cycle with a series of alternating light emissions and pauses;
a light source (14) coupled with the light applicator for supplying the light to the light applicator; and
a controller (16) coupled with the light source for controlling the operation of the light source such that the pauses are timed for movement of the light applicator between light emissions;
wherein the light source (14) is a laser light source; and
the wavelength of the laser light is approximately 1064 nm;
**characterized in that**:
the light emissions of the duty cycle are bursts (22) of laser pulses, the laser pulses having a repetition rate of 10 to 30 hertz.

2. A system (10) according claim 1, wherein there are 10 laser pulses in each burst (22).

3. A system (10) according to claim 1 or claim 2, wherein the time between the end of one burst (22) and the start of the next burst (22) is 0.5 seconds.

4. A system according to claim 3, wherein the duration of each burst (22) is 0.5 seconds.

5. A system (10) according to claim 1 or claim 2, wherein the time between the end of one burst (22) and the start of the next burst (22) is one sixth of a second.

6. A system according to claim 5, wherein the duration of each burst (22) is one third of a second.

7. A system (10) according to any one of the preceding claims, wherein the energy per pulse is 200 millijoules.

8. A system (10) according to any one of the preceding claims, comprising means for providing a light signal and/or an audible tone to signal the completion of a light emission.

## Patentansprüche

1. System (10) zur Behandlung einer Nagelerkrankung bei einem Patienten, wobei das System Folgendes umfasst:
einen Lichtapplikator (12), der betätigbar ist, um eine Nagelerkrankung mit Licht zu bestrahlen, der einen Arbeitszyklus mit einer Reihe abwechselnder Lichtemissionen und Pausen umfasst;
eine Lichtquelle (14), die an den Lichtapplikator gekoppelt ist, um dem Lichtapplikator Licht zuzuführen; und
eine Steuervorrichtung (16), die an die Lichtquelle gekoppelt ist, um den Betrieb der Lichtquelle so zu steuern, dass die Pausen zeitlich auf die Bewegung des Lichtapplikators zwischen Lichtemissionen abgestimmt sind;
worin die Lichtquelle (14) eine Laserlichtquelle ist und die Wellenlänge des Laserlichts etwa 1064 nm beträgt,
**dadurch gekennzeichnet, dass** die Lichtemissionen des Arbeitszyklus Pulsfolgen (22) von Laserimpulsen sind, wobei die Laserimpulse eine Wiederholungsrate von 10 bis 30 Hz aufweisen.

2. System (10) nach Anspruch 1, worin jede Pulsfolge (22) 10 Laserimpulse umfasst.

3. System (10) nach Anspruch 1 oder 2, worin die Zeit zwischen dem Ende einer Pulsfolge (22) und dem Start der nächsten Pulsfolge (22) 0,5 s beträgt.

4. System (10) nach Anspruch 3, worin die Dauer jeder Pulsfolge (22) 0,5 s beträgt.

5. System (10) nach Anspruch 1 oder 2, worin die Zeit zwischen dem Ende einer Pulsfolge (22) und dem Beginn der nächsten Pulsfolge (22) 1/6 einer Sekunde beträgt.

6. System nach Anspruch 5, worin die Dauer jeder Pulsfolge (22) 1/3 einer Sekunde beträgt.

7. System (10) nach einem der vorangegangenen Ansprüche, worin die Energie pro Impuls 200 mJ beträgt.

8. System (10) nach einem der vorangegangenen Ansprüche, das Mittel zur Bereitstellung eines Lichtsignals und/oder eines hörbaren Tons umfasst, um den Abschluss einer Lichtemission zu signalisieren.

## Revendications

1. Système (10) pour le traitement d'une maladie d'ongles chez un patient, le système comprenant :
un applicateur de lumière (12) actionnable pour irradier un ongle atteint d'une maladie d'ongles avec de la lumière comprenant un cycle de travail avec une série d'émissions de lumière et de pauses alternantes ;
une source de lumière (14) couplée à l'applicateur de lumière pour fournir la lumière à l'applicateur de lumière ; et
un dispositif de commande (16) couplé à la source de lumière pour commander le fonctionnement de la source de lumière de telle sorte que les pauses sont synchronisées pour un mouvement de l'applicateur de lumière entre des émissions de lumière ;
où la source de lumière (14) est une source de lumière laser ; et
la longueur d'onde de la lumière laser est approximativement de 1064 nm ;
**caractérisé en ce que** :
les émissions de lumière du cycle de travail sont des salves (22) d'impulsions laser, les impulsions laser ayant un taux de répétition de 10 à 30 hertz.

2. Système (10) selon la revendication 1, où il y a 10 impulsions laser dans chaque salve (22).

3. Système (10) selon la revendication 1 ou la revendication 2, où le temps entre la fin d'une salve (22) et le début de la salve suivante (22) est de 0,5 seconde.

4. Système selon la revendication 3, où la durée de chaque salve (22) est de 0,5 seconde.

5. Système (10) selon la revendication 1 ou la revendication 2, où le temps entre la fin d'une salve (22) et le début de la salve suivante (22) est un sixième d'une seconde.

6. Système selon la revendication 5, où la durée de chaque salve (22) est un tiers d'une seconde.

7. Système (10) selon l'une quelconque des revendications précédentes, où l'énergie par impulsion est de 200 milli-joules.

8. Système (10) selon l'une quelconque des revendications précédentes, comprenant des moyens pour fournir un signal lumineux et/ou un ton audible pour signaler l'achèvement d'une émission de lumière.
